(19) **Europäisches Patentamt / European Patent Office / Office européen des brevets**

(11) **EP 4 685 489 A2**

(12) # EUROPEAN PATENT APPLICATION

(43) Date of publication:
**28.01.2026 Bulletin 2026/05**

(21) Application number: **25221508.2**

(22) Date of filing: **30.07.2024**

(51) International Patent Classification (IPC):
**G01N 33/68** (2006.01)

(52) Cooperative Patent Classification (CPC):
**C07K 16/2803; C07K 16/28; G01N 33/6887; G01N 33/6893;** C07K 2317/34; C07K 2317/76; C07K 2317/92; G01N 2333/4712; G01N 2800/324; G01N 2800/325

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.07.2023 EP 23188639**
**01.11.2023 CN 202311442484**

(62) Document number(s) of the earlier application(s) in accordance with Art. 76 EPC:
**24746369.8 / 4 551 607**

(71) Applicant: **Roche Diagnostics GmbH**
**68305 Mannheim (DE)**

(72) Inventors:
- **Duefel, Hartmut**
  **82377 Penzberg (DE)**
- **Eberl, Magdalena Anna**
  **82377 Penzberg (DE)**
- **Engel, Alfred Michael**
  **82377 Penzberg (DE)**
- **Gerg, Michael**
  **82377 Penzberg (DE)**
- **Hillringhaus, Lars**
  **82377 Penzberg (DE)**
- **Jeske, Tim**
  **82377 Penzberg (DE)**
- **Jucknischke, Ute**
  **82377 Penzberg (DE)**
- **Kastner, Peter**
  **82377 Penzberg (DE)**
- **Meier, Thomas**
  **82377 Penzberg (DE)**
- **Morou, Antigoni**
  **82377 Penzberg (DE)**
- **Speer, Anton**
  **82377 Penzberg (DE)**
- **Wagner, Cornelia**
  **82377 Penzberg (DE)**

(74) Representative: **Altmann Stößel Dick**
**Patentanwälte PartG mbB**
**Theodor-Heuss-Anlage 2**
**68165 Mannheim (DE)**

Remarks:
•The complete document including Reference Table(s) and the Sequence Listing(s) can be downloaded from the EPO website
•This application was filed on 08-12-2025 as a divisional application to the application mentioned under INID code 62.

(54) ## ASSAY FOR MYBPC3

(57) The present invention relates to monoclonal antibodies, or fragments thereof, which bind to MYBPC3 (Myosin binding protein C, cardiac type). Further, the present invention relates to a kit comprising the monoclonal antibodies of the present invention. In some embodiments, the kit is provided as sandwich assay. Further the present invention relates to the in vitro use of the monoclonal antibodies, or fragments thereof, or of the kit of the present invention for the detection of MYBPC3 in a sample. Moreover, the present invention relates to diagnostic methods, such as the assessment of cardiac condition, for example of myocardial infarction.

EP 4 685 489 A2

**Description**

FIELD OF THE INVENTION

The present invention relates to monoclonal antibodies, or fragments thereof, which bind to MYBPC3 (Myosin binding protein C, cardiac type). Further, the present invention relates to a kit comprising the monoclonal antibodies of the present invention. In some embodiments, the kit is provided as sandwich assay. Further the present invention relates to the in vitro use of the monoclonal antibodies, or fragments thereof, or of the kit of the present invention for the detection of MYBPC3 in a sample. Moreover, the present invention relates to diagnostic methods, such as the assessment of cardiac condition, for example of myocardial infarction.

BACKGROUND OF THE INVENTION

An aim of modern medicine is to provide personalized treatment regimens. Those are treatment regimens that take into account a patient's individual needs or risks. Personalized treatment regimens shall be even taken into account for emergency measures where it is required to decide on potential treatment regimens within short periods of time. In such settings, the measurement of circulating biomarkers with a blood test can help to diagnose a disease state (such as injury of myocardial cells with a troponin test).

According to the Universal Definition of Acute Myocardial Infarction (AMI) (Thygesen K et al., Circulation 2018;138:e618-e651. doi: 10.1161/CIR.0000000000000617.), five different types of AMI are defined based on the different mechanistic pathways underlying AMI, such as Type-1 and Type 2 myocardial infarction. The diagnostic workup of patients with suspected AMI (acute myocardial infarction) requires admission to an Emergency Department, registration of a 12-lead electrocardiogram (ECG), a blood test to diagnose or to exclude myocardial injury, assessment of clinical symptoms and history, physical examination, and other diagnostic tests for diagnosis of AMI or differential diagnoses. Diagnosis and management of ACS (Acute Coronary Syndrome) remains challenging for patients that are not ruled-in or ruled-out with high sensitive troponins. Using the 1 hour algorithm with high sensitive troponins between 20 and 25 % of the patients with suspected AMI (Acute Myocardial Infarction) remain in the observe zone which comprises patients which are not triaged towards rule-in/-out after two consecutive measurements at T0 and T1. Using fast protocols (according to the 0 hour algorithm in the ESC (European Society of Cardiology) guidelines) even up to 70 % of patients with suspected AMI remain in the waiting zone (which patients which are not triaged towards rule-in/-out after a single measurement at T0).

Cardiac myosin binding protein-C (MYBPC3, alternative abbreviations: cMyBPC, cMyc or MYBPC3, cMyBP-C) is the cardiac isoform of MyBP and is a structural muscle protein of cardiac myocytes. MYBPC3 has a high cardiac specificity and is released into blood stream following cardiac muscle necrosis. MYBPC3 is a highly specific cardiac protein related to heart muscle contraction. It's specific role and high abundance in heart muscle makes it a superior biomarker candidate for heart muscle damage in circulation. Elevated concentrations of cardiac muscle necrosis biomarkers were described in blood samples of patients with acute myocardial infarction. In addition, MYBPC3 were observed in differential concentrations in type 1 MI versus type 2 MI patients (see e.g. Nestelberger et al., 2021 JAMA Cardiol. doi:10.1001/jamacardio.2021.0669).

Kozhuharov et al. disclose that MYBPC3 can be used in the diagnosis and risk stratification of acute heart failure (Eur J Heart Fail. 2021 May;23(5):716-725. doi: 10.1002/ejhf.2094. Epub 2021 Feb 5. PMID: 33421273).

Kaier et al. disclose an algorithm using cardiac myosin-binding protein C for early diagnosis of myocardial infarction (European Heart Journal. Acute Cardiovascular Care, Volume 11, Issue 4, April 2022, Pages 325-335).

WO 2015/110114 and WO2015/110115 disclose monoclonal antibodies that bind MYBPC3.

Marjot et al. describe the development and application of a high-sensitivity immunoassay for cardiac myosin-binding protein C. Transl Res 2016;170:17-25.e5.

However, MYBPC3 assays which allow the detection of the biomarker with high sensitivity are still needed. The aim of the present invention was therefore to provide means and methods which allow for a reliable detection of MYBPC3.

The inventors have identified two antibodies, designated and mAB "7B5" and mAB "4.1.1" that are particularly useful for the detection of MYBPC3. The antibodies 7B5 and M-4.1.1 recognize non-linear, conformational epitopes presented by the natively folded MYBPC3 protein (see Tables 7 and 8 in the Examples section). In particular, the antibodies can be used as an antibody pair in a sandwich assay. The sandwich assay of the present invention allow for an assessment of cardiac conditions, such as myocardial infarction or heart failure.

BRIEF SUMMARY OF THE PRESENT INVENTION

The present invention relates to a monoclonal antibody, or fragment thereof, which binds to MYBPC3 (Myosin binding protein C, cardiac type).

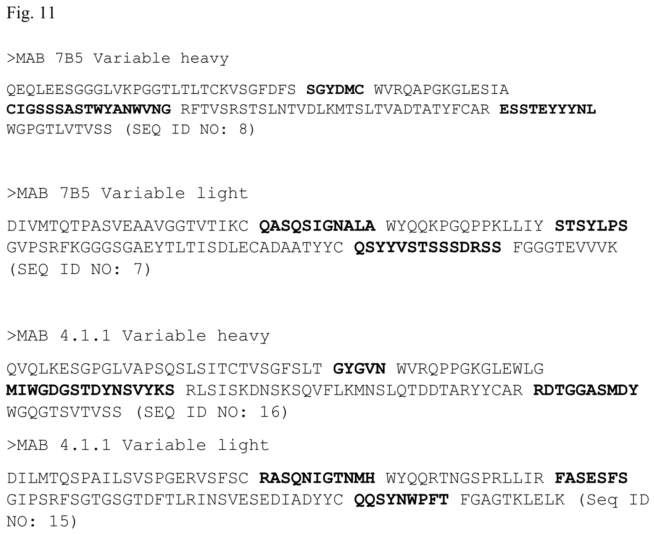

In a first aspect, the present invention relates to first monoclonal antibody, or fragment thereof, which binds to MYBPC3 (Myosin binding protein C, cardiac type). The first antibody, or fragment thereof, as referred herein is preferably the antibody designated 7B5, or an antigen binding fragment thereof. The amino acid sequences of the light and heavy chain variable domains and of the CDR sequences of the antibody designated 7B5 are shown in Figure 11. However, the first monoclonal antibody, or fragment thereof, is not limited to this antibody. Further encompassed by the present invention are variants of this antibody, or fragment thereof, provided that the variant binds MYBPC3.

In a preferred embodiment, the first monoclonal antibody, or fragment thereof, comprises a first light chain variable domain and/or a first heavy chain variable domain,

(a) said first light chain variable domain comprising

(a1) a first light chain CDR1 that differs by not more than a total of three amino acid additions, substitutions, and/or deletions from said first light chain CDR1 having an amino acid sequence as shown in SEQ ID NO: 1 (QASQSIGNALA),
(a2) a first light chain CDR2 that differs by not more than a total of three amino acid additions, substitutions, and/or deletions from said first light chain CDR2 having an amino acid sequence as shown in SEQ ID NO: 2 (STSYLPS), and/or
(a3) a first light chain CDR3 that differs by not more than a total of three amino acid additions, substitutions, and/or deletions from said first light chain CDR3 having an amino acid sequence as shown in SEQ ID NO: 3 (QSYYVSTSSSDRSS),

(b) said first heavy chain variable domain comprising

(b1) a first heavy chain CDR1 that differs by not more than a total of three amino acid additions, substitutions, and/or deletions from said first heavy chain CDR1 having an amino acid sequence as shown in SEQ ID NO: 4 (SGYDMC), (b2) a first heavy chain CDR2 that differs by not more than a total of three amino acid additions, substitutions, and/or deletions from said first heavy chain CDR2 having an amino acid sequence as shown in SEQ ID NO: 5 (CIGSSSASTWYANWVNG), and/or
(b3) a first heavy chain CDR3 that differs by not more than a total of three amino acid additions, substitutions, and/or deletions from said first heavy chain CDR3 having an amino acid sequence as shown in SEQ ID NO: 6 (ESSTEYYYNL).

In a preferred embodiment, the first monoclonal antibody, or fragment thereof, comprises

(a) a first light chain variable domain comprising

(a1) said first light chain CDR1 that differs by not more than one amino acid addition, substitution or deletion from said first light chain CDR1 having an amino acid sequence as shown in SEQ ID NO: 4 (QASQSIGNALA),
(a2) said first light chain CDR2 that differs by not more than one amino acid addition, substitution or deletion from said first light chain CDR2 having an amino acid sequence as shown in SEQ ID NO: 5 (STSYLPS), and/or
(a3) said first light chain CDR3 that differs by not more than one amino acid addition, substitution or deletion from said first light chain CDR3 having an amino acid sequence as shown in SEQ ID NO: 6 (QSYYVSTSSSDRSS),

(b) a first heavy chain variable domain comprising

(b1) a first heavy chain CDR1 that differs by not more than one amino acid addition, substitution or deletion from a first heavy chain CDR1 having an amino acid sequence as shown in SEQ ID NO: 7 (SGYDMC),
(b2) a first heavy chain CDR2 that differs by not more than one amino acid addition, substitution or deletion from a first heavy chain CDR2 having an amino acid sequence as shown in SEQ ID NO: 8 (CIGSSSASTWYANWVNG), and/or
(b3) a first heavy chain CDR3 that differs by not more than one amino acid addition, substitution or deletion from a first heavy chain CDR3 having an amino acid sequence as shown in SEQ ID NO: 9 (ESSTEYYYNL),

In a particularly preferred embodiment, the first monoclonal antibody, or fragment thereof comprises

(a) a first light chain variable domain comprising

(a1) said first light chain CDR1 having a sequence as shown in SEQ ID NO: 1 (QASQSIGNALA),
(a2) said first light chain CDR2 having a sequence as shown in SEQ ID NO: 2 (STSYLPS), and

(a3) said first light chain CDR3 having a sequence as shown in SEQ ID NO: 3 (QSYYVSTSSSDRSS),

and
(b) a first heavy chain variable domain comprising

(b1) a first heavy chain CDR1 having a sequence as shown in SEQ ID NO: 4 (SGYDMC),
(b2) a first heavy chain CDR2 having a sequence as shown in SEQ ID NO: 5 (CIGSSSASTWYANWVNG), and
(b3) a first heavy chain CDR3 having a sequence as shown in SEQ ID NO: 6 (ESSTEYYYNL).

Typically, the first monoclonal antibody, or fragment thereof, comprises a first light chain variable domain and/or a first heavy chain variable domain, wherein

said first light chain variable domain is at least 85%, such as at least 90% or at least 95% identical to the light chain variable domain having a sequence as shown in SEQ ID NO: 7:

DIVMTQTPASVEAAVGGTVTIKC QASQSIGNALA WYQQKPGQPPKLLIY STSYLPS GVPSRFKGGGSGAEYTLTISDLECADAATYYC QSYYVSTSSSDRSS FGGGTEVVVK, and

the first heavy chain variable domain is at least 85% identical, such as at least 90% or at least 95% identical to the heavy chain variable domain having a sequence as shown in SEQ ID NO: 8: QEQLEESGGGLVKPGGTLTLTCKVSGFDFS SGYDMC WVRQAPGKGLESIA CIGSSSASTWYANWVNG RFTVSRSTSLNTVDLKMTSLTVADTATYFCAR ESSTEYYYNL WGPGTLVTVSS.

In a preferred embodiment of the first monoclonal antibody or fragment thereof, the first light chain variable domain comprises a sequence as shown in SEQ ID NO: 7, and the first heavy chain variable domain comprises a sequence as shown in SEQ ID NO: 8.

In a second aspect, the present invention relates to second monoclonal antibody, or fragment thereof, which binds to MYBPC3 (Myosin binding protein C, cardiac type).

The second antibody, or fragment thereof, as referred herein is preferably the antibody designated 4.1.1, or an antigen binding fragment thereof. However, the second monoclonal antibody, or fragment thereof, is not limited to this antibody. Further encompassed by the present invention are variants of the antibody 4.1.1, or fragment thereof, provided that the variant binds MYBPC3.

The amino acid sequences of the light and heavy chain variable domains and of the CDR sequences of the antibody designated 4.1.1 are shown in Figure 11.

In an embodiment, the second monoclonal antibody, or fragment thereof, comprises a second light chain variable domain and/or a second heavy chain variable domain, wherein

(a) said second light chain variable domain comprising

(a1) a second light chain CDR1 that differs by not more than a total of three amino acid additions, substitutions, and/or deletions from a second light chain CDR1 having an amino acid sequence as shown in SEQ ID NO: 9 (RASQNIGTNMH),
(a2) a second light chain CDR2 that differs by not more than a total of three amino acid additions, substitutions, and/or deletions from a second light chain CDR2 having an amino acid sequence as shown in SEQ ID NO: 10 (FASESFS), and/or
(a3) a second light chain CDR3 that differs by not more than a total of three amino acid additions, substitutions, and/or deletions from a second light chain CDR3 having an amino acid sequence as shown in SEQ ID NO: 11 (QQSYNWPFT),

(b) said second heavy chain variable domain comprising

(b1) a second heavy chain CDR1 that differs by not more than a total of three amino acid additions, substitutions, and/or deletions from said second heavy chain CDR1 having an amino acid sequence as shown in SEQ ID NO: 12 (GYGVN),
(b2) a second heavy chain CDR2 that differs by not more than a total of three amino acid additions, substitutions, and/or deletions from said second heavy chain CDR2 having an amino acid sequence as shown in SEQ ID NO: 13 (MIWGDGSTDYNSVYKS), and/or

(b3) a second heavy chain CDR3 that differs by not more than a total of three amino acid additions, substitutions, and/or deletions from said second heavy chain CDR3 having an amino acid sequence as shown in SEQ ID NO: 14 (RDTGGASMDY).

In a preferred embodiment, the second monoclonal antibody, or fragment thereof, comprises

(a) a second light chain variable domain comprising

(a1) a second light chain CDR1 that differs by not more than one amino acid addition, substitution or deletion from a second light chain CDR1 having an amino acid sequence as shown in SEQ ID NO: 9 (RASQNIGTNMH),
(a2) a second light chain CDR2 that differs by not more than one amino acid addition, substitution or deletion from a second light chain CDR2 having an amino acid sequence as shown in SEQ ID NO: 10 (FASESFS), and/or
(a3) a second light chain CDR3 that differs by not more than one amino acid addition, substitution or deletion from a second light chain CDR3 having an amino acid sequence as shown in SEQ ID NO: 11 (QQSYNWPFT),

(b) a second heavy chain variable domain comprising

(b1) a second heavy chain CDR1 that differs by not more than one amino acid addition, substitution or deletion from a second heavy chain CDR1 having an amino acid sequence as shown in SEQ ID NO: 12 (GYGVN),
(b2) a second heavy chain CDR2 that differs by not more than one amino acid addition, substitution or deletion from a second heavy chain CDR2 having an amino acid sequence as shown in SEQ ID NO: 13 (MIWGDGSTDYNSVYKS), and/or
(b3) a second heavy chain CDR3 that differs by not more than one amino acid addition, substitution or deletion from a second heavy chain CDR3 having an amino acid sequence as shown in SEQ ID NO: 14 (RDTGGASMDY).

In a particularly preferred embodiment, the second monoclonal antibody, or fragment thereof, comprises

(a) a second light chain variable domain comprising

(a1) a second light chain CDR1 having a sequence as shown in SEQ ID NO: 9 (RASQNIGTNMH),
(a2) a second light chain CDR2 having a sequence as shown in SEQ ID NO: 10 (FASESFS), and
(a3) a second light chain CDR3 having a sequence as shown in SEQ ID NO: 11 (QQSYNWPFT),

and
(b) a second heavy chain variable domain comprising

(b1) a second heavy chain CDR1 having a sequence as shown in SEQ ID NO: 12 (GYGVN),
(b2) a second chain CDR2 having a sequence as shown in SEQ ID NO: 13 (MIWGDGSTDYNSVYKS), and
(b3) a second heavy chain CDR3 having a sequence as shown in SEQ ID NO: 14 (RDTGGASMDY).

Typically, the second monoclonal antibody, or fragment thereof, comprises a second light chain variable domain and/or a second heavy chain variable domain, wherein said second light chain variable domain is at least 85%, such as at least 90% or at least 95% identical to the light chain variable domain having a sequence as shown in SEQ ID NO: 15 (DILMTQSPAILSVSPGERVSFSC RASQNIGTNMH WYQQRTNGSPRLLIR FASESFS GIPSRFSGTGSGTDFTLRINSVESEDIADYYC QQSYNWPFT FGAGTKLELK), and a second heavy chain variable domain that is at least 85%, such as at least 90% or at least 95% identical to the heavy chain variable domain having a sequence as shown in SEQ ID NO: 16 (QVQLKESGPGLVAPSQSLSITCTVSGFSLT     GYGVN     WVRQPPGKGLEWLG     MIWGDGSTDYNSVYKS RLSISKDNSKSQVFLKMNSLQTDDTARYYCAR RDTGGASMDY WGQGTSVTVSS).

In a preferred embodiment of the second monoclonal antibody or fragment thereof, the second light chain variable domain comprises a sequence as shown in SEQ ID NO: 15, and the second heavy chain variable domain comprises a sequence as shown in SEQ ID NO: 16.

The term "fragment" as used herein, typically, refers to an "antigen-binding fragment". In a preferred embodiment, the fragment of the antibody as referred to herein is Fab fragment, a Fab' fragment, a Facb fragment, a F(ab')2 fragment, a scFv fragment, or a Fv fragment, in particular the fragment is a F(ab')2 fragment. In an embodiment, the fragment is a F(ab')2 fragment.

In a preferred embodiment of the monoclonal antibody, or fragment thereof, of the present invention the antibody is labelled. In an embodiment, the monoclonal antibody (or fragment thereof) is biotinylated. In another embodiment, the monoclonal antibody (or fragment thereof) is ruthenylated. Accordingly, the antibody (or an antigen-binding fragment thereof) shall comprise a ruthenium label. In an embodiment, said ruthenium label is a bipyridine-ruthenium(II) complex. Alternatively the antibody (or an antigen-binding fragment thereof) shall comprise an iridium label. In an embodiment, said iridium label is a complex as disclosed in WO 2012/107419.

In a further aspect, the present invention relates to kit comprising the first antibody, or fragment thereof, and the second antibody, or fragment thereof, of the present invention.

In an embodiment of the kit of the present invention, the first antibody, or fragment thereof, and the second antibody, or fragment thereof, are provided as sandwich assay. Thus, the kit of the present invention preferably is a sandwich assay. Preferably, the first monoclonal antibody (or fragment thereof) is biotinylated and the second monoclonal antibody (or fragment thereof) is ruthenylated.

In a preferred embodiment, the kit comprises a biotinylated F(ab')2 fragment of the first monoclonal antibody as referred to herein and a second monoclonal antibody of the present invention, said second monoclonal antibody comprising an electrochemiluminescent label. The two antibodies form sandwich immunoassay complexes with the MYBPC3 polypeptide in the sample.

In an embodiment, the kit further comprises the MYBPC3 polypeptide, or a fragment thereof (e.g. as a control and/or calibrator).

In an embodiment, the present invention relates to the in vitro use of the kit of the present invention for the quantitative determination of cardiac myosin binding protein C (cMyBP-C) in human serum and plasma.

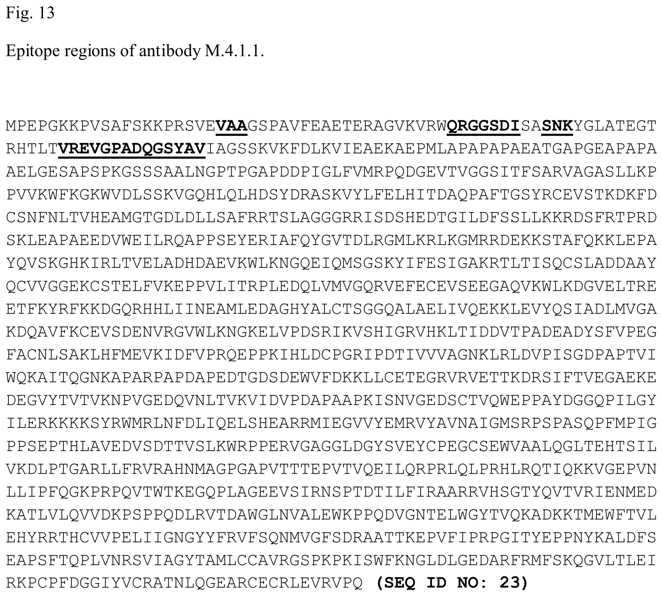

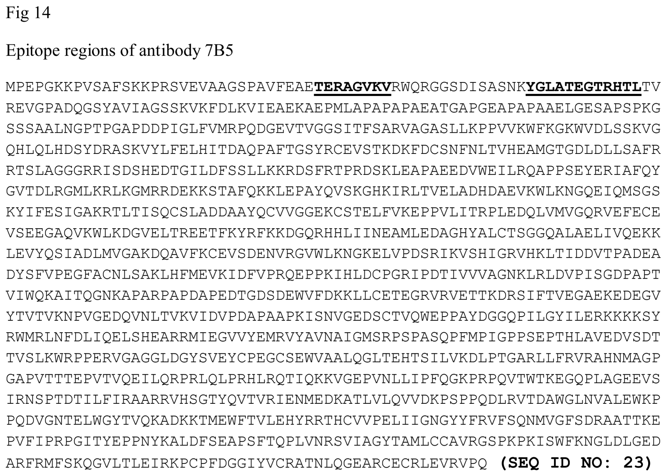

The amino acid sequence of the full-length MYBPC3 polypeptide is shown in SEQ ID NO: 23. However, the kit is not limited to the full-length polypeptide. Rather, a kit may comprise a fragment of the full-length polypeptide, provided that is comprises the epitope binding regions of the antibody of the present invention (see Tables 7 and 8, and Figures 13 and 14). In an embodiment, the fragment comprises an amino acid sequence as shown in SEQ ID NO:17 or 18. As compared to the sequence shown in SEQ ID NO:18, SEQ ID NO:17 further comprises a His-Tag.

In a further aspect, the present invention relates the in vitro use of the monoclonal antibody, or fragment thereof, of the present invention or of the kit of the present invention for the detection of MYBPC3 in a sample. In some embodiment, the detection is the quantitative detection.

In a preferred embodiment of the use of method of present invention, the sample is a body fluid, such as a blood, serum or plasma sample.

The present invention further concerns a method for detecting MYBPC3 in a sample, comprising

> (a) contacting a sample comprising MYBPC3 with the first and/or second monoclonal antibody, or fragment thereof, of the present invention, thereby forming a complex comprising MYBPC3 and said monoclonal antibody, or fragment thereof (or said monoclonal antibodies or fragments thereof), and
>
> (b) detecting the complex formed in step (a), thereby detecting MYBPC3 in said sample.

The present invention further pertains to a complex comprising the first and/or second monoclonal antibody, or fragment thereof, of the present invention and MYBPC3. Preferably, the complex comprises the following:

> i) the MYBPC3 polypeptide
> ii) the first monoclonal antibody, or a fragment thereof (e.g. in biotinylated from), and
> iii) the second monoclonal antibody, or a fragment thereof (e.g. in ruthenylated from).

The present invention further pertains relates to the in vitro use of the monoclonal antibody, or fragment thereof, of the present invention, or of the kit of the present invention for assessing a cardiac condition.

In a preferred embodiment, the cardiac condition is heart failure.

In another preferred embodiment, the cardiac condition is myocardial injury, in particular myocardial infarction.

In a preferred embodiment of the in vitro use of the present invention, myocardial infarction is ruled out.

In another preferred embodiment of the in vitro use of the present invention, it is differentiated between Type 1 and Type 2 myocardial infarction.

In another preferred embodiment of the in vitro use of the present invention, the risk for of myocardial infarction and/or cardiovascular death at 30 days for patients suspected of acute coronary syndrome (ACS) is predicted, for example the risk at 30 days.

The Figures show

Fig. 1:     Purified MYBPC3 was characterized by SDS Page and SEC-RALS HPLC

Fig. 2     ELISA of supernatants from B-cell- or mouse hybridoma-cultivation

(a) 96/384-well MTPs, streptavidin coated
(b) recombinant MYBPC3, monomeric, biotinylated via Avi-tag
(c) mAb samples (supernatants of B-cell- or hybridoma-cultivations)
(d) anti-rabbit (anti-mouse)-IgG POD-conjugate

Fig. 3:      Kinetic profiles for antibodies from mouse supernatants binding MYBPC3 at 37°C. (A) Antibody M-4.000.1 was selected for its fast and stable MYBPC3 complex formation with a suitable half-life time t/2 diss of> 23 minutes (left). The dissociation fit (light grey) is overlaid to the measured kinetic profile (right). (B) Exemplary kinetic profiles of deselected antibodies with either slow MYBPC3 complex formation, fast dissociation or complex binding behavior - not obeying Langmuir Law or artifact binding.

Fig. 4:      Kinetic characterization for antibodies binding MYBPC3 at 37°C. Ab-Interactions with increasing MYBPC3 concentrations, c = 0.4-90 nM for antibody 7B5 (A) resp. M-4.1.1 (B). Shown are 2 independent concentration series (black) with a duplicate for concentration 30 nM for each of the antibodies, overlaid by a Langmuir 1:1 fitting model, Rmax global, RI = 0 (grey). The first concentration series is shown with a 30 minutes monitored dissociation phase (left overlay); Both series "zoomed in" with shown 10 minutes dissociation phase (middle and right).

Fig. 5       Overlay for immune MYBPC3 complex with antibody pair 4.1.1 and 7B5. Grey arrows up and down mark the start respectively the end of the injections (1) MYBPC3, (2) secondary antibody, here 7B5

Fig. 6:      Correlation between Roche and Erenna MYBPC3 measurements. The correlation between the two assays is high (log-transformed, Pearson correlation = 0.894).

Fig. 7:      Outcome clinical performance (Diagnosis of heart failure). The AUC ("Area under Curve") of the Roche MYBPC3 Assay is higher compared to the Erenna Assay (see plot).

Fig. 8:      Outcome correlation: For in total 1056 patients, Roche and Erenna MYBPC3 measurements were matched. The correlation between the two assays is high (P earson correlation = 0.9726981).

Fig. 9:      Diagnosis of MI: The AUC of the MYBPC3 Assay of the present invention is higher compared to the Erenna Assay. Using a one sided DeLong test to compare the AUC's shows significance (p-value = 0.02269). Similar performance in the rule in (left-bottom) and slightly better performance in the rule out (right-up) zone was observed.

Fig. 10:     Distinguishing between Type 1 and Type 2 MI: In total we have 52 Type 2 and 153 Type 1. The AUC is higher for the Roche Assay. The DeLong test shows no significance (p value = 0.1387).

Fig. 11      Amino acid sequences of the variable domains (heavy and light) of the monoclonal antibodies 7B5 and 4.1.1. The CDR sequences are shown in bold.

Fig. 12      Risk prediction for Major Adverse Cardiovascular Events (MACE) within 30 days. The AUC values for the assay of the present invention and the Erenna assay are shown.

Fig. 13      Epitope regions of antibody M.4.1.1. The binding regions of the antibody are highlighted in bold and underlined.

Fig. 14      Epitope regions of antibody 7B5. The binding regions of the antibody are highlighted in bold and underlined.

DETAILED SUMMARY OF THE PRESENT INVENTION - DEFINITIONS

As set forth above, the present invention relates to monoclonal antibody, or fragments thereof, which binds to MYBPC3 (Myosin binding protein C, cardiac type), in particular to human MYBPC3.

MYBPC3 (or MYBPC3) is produced in heart muscle. It is a 140.5 kDa protein composed of 1273 amino acids MYBPC3 is a myosin-associated protein that binds at 43 nm intervals along the myosin thick filament backbone, stretching for 200 nm on either side of the M-line within the crossbridge-bearing zone (C-region) of the A band in striated muscle. The MYBPC3 is encoded by the MYBPC3 gene. Further information on human MYBPC3 can be found in the UniProtKB database under accession number Q14896 (MYPC3_HUMAN). The full length amino acid sequence is shown in SEQ ID NO:23 as well as in Figures 13 and 14.

It is to be understood that as used in the specification and in the claims, "a" or "an" can mean one or more, depending upon the context in which it is used. Thus, for example, reference to "an" item can mean that at least one item can be utilized.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. The term "comprising" also encompasses embodiments where only the items referred to are present, i.e. it has a limiting meaning in the sense of "consisting of".

Further, as used in the following, the terms "particularly", "more particularly", "typically", and "more typically" or similar terms are used in conjunction with additional / alternative features, without restricting alternative possibilities. Thus, features introduced by these terms are additional / alternative features and are not intended to restrict the scope of the claims in any way. The invention may, as the skilled person will recognize, be performed by using alternative features. Similarly, features introduced by "in an embodiment of the invention" or similar expressions are intended to be additional / alternative features, without any restriction regarding alternative embodiments of the invention, without any restrictions regarding the scope of the invention and without any restriction regarding the possibility of combining the features introduced in such way with other additional / alternative or non-additional / alternative features of the invention.

Further, it will be understood that the term "at least one" as used herein means that one or more of the items referred to following the term may be used in accordance with the invention. For example, if the term indicates that at least one sampling unit shall be used this may be understood as one sampling unit or more than one sampling units, i.e. two, three, four, five or any other number. Depending on the item the term refers to, the skilled person understands as to what upper limit the term may refer, if any.

The term "about" as used herein means that with respect to any number recited after said term an interval accuracy exists within in which a technical effect can be achieved. Accordingly, "about" as referred to herein, preferably, refers to the precise numerical value or a range around said precise numerical value of $\pm 20$ %, preferably +15 %, more preferably $\pm 10$ %, or even more preferably $\pm 5$ %.

The term "subject" as used herein refers to an animal, preferably a mammal and, more typically to a human. The subject to be investigated by the method or use of the present invention shall be suspected to suffer from cardiac condition or shall suffer from cardiac condition. In an embodiment, the cardiac condition is myocardial infarction.

The term "sample" as used herein refers to any sample that under physiological conditions comprises the first, second and/or third biomarkers referred to herein. More typically, the sample is a body fluid sample, e.g. a blood sample or sample derived therefrom (e.g. serum or plasma), a urine sample, a saliva sample, interstitial fluid, a lymphatic fluid sample or the like. In a preferred embodiment, the sample is a blood (i.e. whole blood), serum or plasma sample. Serum is the liquid fraction of whole blood that is obtained after the blood is allowed to clot. For obtaining the serum, the clot is removed by centrifugation and the supernatant is collected. Plasma is the acellular fluid portion of blood. For obtaining a plasma sample, whole blood is collected in anticoagulant-treated tubes (e.g. citrate-treated or EDTA-treated tubes). Cells are removed from the sample by centrifugation and the supernatant (i.e. the plasma sample) is obtained.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity. Preferably, standard parameters are applied for determining the degree of sequence identity of two sequences. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology aligmnent algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. In an embodiment, the percent identity between two amino acid sequences is determined using the Needleman and Wunsch algorithm (Needleman 1970, J. Mol. Biol. (48):444-453) which has been incorporated into the needle program in the EMBOSS software package (EMBOSS: The European Molecular Biology Open Software Suite, Rice, P., Longden, I., and Bleasby, A., Trends in Genetics 16(6), 276-277, 2000), a BLOSUM62 scoring matrix, and a gap opening penalty of 10 and a gap entension pentalty of 0.5. A preferred, non-limiting example of parameters to be used for aligning two amino acid sequences using the needle program are the default parameters, including the EBLOSUM62 scoring matrix, a gap opening penalty of 10 and a gap extension penalty of 0.5.

The term "antibody" refers to immunoglobulins or immunoglobulin-like molecules including by way of example and without limitation, IgA, IgD, IgE, IgG and IgM, combinations thereof, and similar molecules produced during an immune response in any vertebrate, for example, in mammals such as goats, rabbits and mice, as well as non-mammalian species, such as shark immunoglobulins. For example, the antibody may be a rabbit antibody. The term "antibody" includes intact immunoglobulins and "antibody fragments" or "antigen binding fragments" that specifically bind to a molecule of interest (or a group of highly similar molecules of interest) to the substantial exclusion of binding to other molecules. The term "antibody" also includes genetically engineered forms such as chimeric antibodies (for example, humanized murine antibodies), heteroconjugate antibodies (such as, bispecific antibodies). See also, Pierce Catalog and Handbook, 1994-1995 (Pierce Chemical Co., Rockford, 111.); Kuby, J., Immunology, 3rd Ed., W.H. Freeman & Co., New York, 1997.

In particular, the term "antibody" refers to a polypeptide ligand comprising at least a light chain and heavy chain immunoglobulin variable region which specifically recognizes and binds an epitope of an antigen. Antibodies are composed of a heavy and a light chain, each of which has a variable region, termed the variable heavy (VH) region and the variable light (VL) region. Together, the VH region and the VL region are responsible for binding the antigen recognized by the antibody. Typically, the antibody of the present invention has heavy (H) chains and light (L) chains interconnected by disulfide bonds. The term "light chain" as used herein includes a full-length light chain and fragments thereof having a sufficient variable region sequence to confer binding specificity. A full-length light chain includes a variable region domain, $V_L$, and a constant region domain, $C_L$. The variable region domain of the light chain is at the amino-terminus of the polypeptide. Light chains include kappa chains and lambda chains. The term "heavy chain" includes a full-length heavy chain and fragments thereof having sufficient variable region sequence to confer binding specificity. A full-length heavy chain includes a variable region domain, $V_H$, and three constant region domains, $C_H1$, $C_H2$, and $C_H3$, The $V_H$ domain is at the amino-terminus of the polypeptide, and the $C_H$ domains are at the carboxyl-terminus, with the $C_H3$ being closest to the carboxy-terminus of the polypeptide,

There are five main heavy chain classes (or isotypes) which determine the functional activity of an antibody molecule: IgM, IgD, IgG, IgA and IgE. Each heavy and light chain contains a constant region and a variable region (the regions are also known as "domains"). In combination, the heavy and the light chain variable regions specifically bind the antigen. Light and heavy chain variable regions contain a "framework" region interrupted by three hypervariable regions, also called "complementarity-determining regions" or "CDRs". The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a VH CDR3 is located in the variable domain of the heavy chain of the antibody in which it is found, whereas a VL CDR1 is the CDR1 from the variable domain of the light chain of the antibody in which it is found. An antibody that binds MYBPC3 will have a specific VH region and the VL region sequence, and thus specific CDR sequences.

Complementarity determining regions (CDRs) and framework regions of a given antibody can be identified using the system described by Kabat et al., in Sequences of Proteins of Immunological Interest, 5th Ed., US Dept. of Health and Human Services, PHS, NIH, NIH Publication no. 91-3242, 1991. In the studies underlying the present invention, CDR sequences were determined according to the system described by Kabat. However, the CDRs can also be redefined according to an alternative nomenclature scheme based on IMGT definition (Lefranc, M.P. et al., 2003, Dev Comp Immunol. 27(1): 55-77).

The antibody, or fragment thereof, of the present invention may be a single chain antibody, an IgD antibody, an IgE antibody, an IgM antibody, an IgG antibody and fragments thereof. For example, the antibody may be an IgG antibody such as an IgG1 antibody; an IgG2 antibody; an IgG3 antibody; or an IgG4 antibody. In an embodiment, the antibody, or fragment thereof, has been produced recombinantly.

Also encompassed by the present invention are fragments of the monoclonal antibody of the present invention. The fragment shall be an immunologically functional fragment, i.e. an antigen (MYBPC3)-binding fragment. Accordingly, the fragment of the monoclonal antibody of the present invention shall be capable of the binding MYBPC3, such as human MYBPC3. Accordingly, the term "immunologically functional fragment" of an antibody, as used herein, refers to a portion of an antibody that lacks at least some of the amino acids present in a full-length chain but which is capable of specifically binding to MYBPC3. Immunologically functional immunoglobulin fragments include Fab, Fab', F(ab')$_2$, and Fv fragments. How to produce antigen-binding fragments is well-known in the art. For example, the fragments can be produced by enzymatic cleavage of an antibody of the present invention. In addition, the fragments can be generated by synthetic or recombinant techniques. Fab fragments are preferably generated by papain digestion of an antibody, Fab' fragments by pepsin digestion and partial reduction, F(ab')$_2$ fragments by pepsin digestion. Fv fragments are preferably produced by molecular biology techniques.

The fragment of an antibody may also be a diabody, which are small antibody fragments with two antigen-binding sites. Diabodies preferably comprise a heavy chain variable domain connected to a light chain variable domain in the same polypeptide chain.

The antibody of the present invention is preferably a monoclonal antibody. As used herein, the term "monoclonal antibody" refers to an antibody produced by a single clone of B-lymphocytes or by a cell into which the light and heavy chain genes of a single antibody have been transfected. Monoclonal antibodies are produced by methods known to those of skill in the art, for instance by making hybrid antibody-forming cells from a fusion of myeloma cells with immune spleen cells.

In an embodiment, the antibody of the present invention is an isolated antibody. Thus, the antibody shall be an antibody which has been purified. Purification of an antibody can be achieved by methods well-known in the art such as Size Exclusion Chromatography (SEC). Accordingly, the antibody shall have been isolated from the cells in which the antibody was produced. In some embodiments, an isolated antibody is purified to greater than 70% by weight of antibody as determined by, for example, the Lowry method, and in some embodiments, to greater than 80%, 90%, 95%, 96%, 97%, 98% or 99% by weight. In one preferred embodiment the isolated antibody according to the present invention is purified to greater than 90% purity as determined by SDS-PAGE under reducing conditions using Coomassie blue staining for protein detection.

The monoclonal antibody of the present invention, or fragment thereof, shall specifically bind to the MYBPC3 polypeptide, e.g. to the human MYBPC3 polypeptide. The expression "binding" and "specifically binding" are well understood and are used to indicate that an antibody (or fragment thereof) does not significantly bind to other biomolecules.

Preferably, the antibody of the present invention (or fragment thereof) shall specifically bind to the natively folded MYBPC3 protein. In particular, they shall recognize a non-linear, conformational epitope presented by the natively folded MYBPC3 protein. The amino acid residues comprised by the conformational epitopes of antibodies 7B5 and M-4.1.1 are shown in Tables 7 and 8, respectively. The epitopes are present at the N-terminal portion of the MYBPC3 protein. The epitope regions are also highlighted in Figures 13 and 14. Typically, the antibody of the present invention binds the epitope regions shown in the aforementioned tables and figures.

The term "epitope" denotes a protein determinant capable of specifically binding to an antibody. Thus, the term preferably refers to the portion of the MYBPC3 polypeptide (such as the human MYBPC3 polypeptide) capable of being specifically bound by the antibody of the present invention (or the fragment thereof). Epitopes usually consist of chemically active surface groupings of molecules such as amino acids and usually epitopes have specific three-dimensional structural characteristics, as well as specific charge characteristics.

In a preferred embodiment, the antibody of the present invention or the fragment thereof is linked to a detectable label. A detectable label as described herein is preferably a label which is not naturally linked to an antibody or antigen-binding fragment thereof. Thus, the detectable label is preferably heterologous with respect to the antibody. Suitable labels are any labels detectable by an appropriate detection method. In an embodiment said detectable label is an enzyme, biotin, a radioactive label, a fluorescent label, a chemiluminescent label, an electrochemiluminescent label, a gold label, or a magnetic label.

Enzymatic labels include e.g. horseradish peroxidase, alkaline phosphatase, beta-galactosidase, and luciferase. The substrates for these enzymes are well-known in the art. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate). A suitable enzyme-substrate combination may result in a colored reaction product, fluorescence or chemoluminescence, which can be measured according to methods known in the art. Fluorescent labels, e.g., include 5-carboxyfluorescein, fluorescein isothiocyanate, rhodamine, tetramethylrhodamine, Cy2, Cy3, and Cy5, fluorescent proteins such as GFP (Green Fluorescent Protein), Texas Red and the Alexa dyes. Radioactive labels, e.g., include radioactive isotopes of iodide, cobalt, selenium, tritium, carbon, sulfur and phosphorous. A radioactive label can be detected by any method known and appropriate, e.g. a light-sensitive film or a phosphor imager. Magnetic labels e.g. include paramagnetic and superparamagnetic labels. Chemiluminescent labels of use may include luminol, isoluminol, an aromatic acridinium ester, an imidazole, an acridinium salt or an oxalate ester.

The present invention also relates to a host cell producing the antibody of the present invention, or the antigen-binding fragment thereof. In a preferred embodiment, the host producing the antibody of the present invention is a hybridoma cell. Moreover, the host cell may be any kind of cellular system which can be engineered to generate the antibodies according to the current invention. For example, the host cell may be an animal cell, in particular a mammalian cell. In one embodiment HEK293 (human embryonal kidney cells) such as HEK 293-F cells as used in the Examples section, or CHO (Chinese hamster ovary) cells are used as host cells. In another embodiment, the host cell is a non-human animal or mammalian cell.

The host cell preferably comprises at least one polynucleotide encoding for the antibody of the present invention, or fragment thereof. For example, the host cell comprises at least one polynucleotide encoding for the light chain of the antibody of the present invention and at least one polynucleotide encoding the heavy chain of the antibody of the present invention. Said polynucleotide(s) shall be operably linked to a suitable promoter.

The definition and explanations given herein above, preferably, apply *mutatis mutandis* to the following.

The antibody of the present invention or antigen-binding fragment thereof is useful in methods relating to the localization and/or quantitation of MYBPC3 polypeptide. For example, the antibody, or fragment thereof, allows for determining the amount of the MYBPC3 polypeptide in a sample, for use in diagnostic methods, or for imaging the MYBPC3 polypeptide.

Accordingly the present invention relates to *the vitro* use of the monoclonal antibody, or fragment thereof, of the present invention, or of the kit of the present invention for the detection of MYBPC3, i.e. of the MYBPC3 polypeptide in a sample. In an embodiment, the detection is the quantitative detection. Typically, the sample is a body fluid, such as a blood, serum or plasma sample. Typically, the sample is derived from a subject described herein elsewhere.

Moreover, the present invention relates to a method for detecting MYBPC3 in a sample, comprising

(a) contacting a sample comprising MYBPC3 with the first and/or second monoclonal antibody, or fragment thereof, of the invention, thereby forming a complex comprising MYBPC3 and said monoclonal antibody (or both monoclonal antibodies), or fragment (s) thereof, and
(b) detecting the complex formed in step (a), thereby detecting MYBPC3 in said sample.

Preferably, the detection is the determination of the amount of MYBPC3 in the sample.

Preferably, the sample in step a) of the above method is contacted with, the first and second monoclonal antibody, or fragment thereof, of the invention, thereby forming a complex comprising MYBPC3 and both monoclonal antibodies, or antigen-binding fragments thereof.

For the detection of MYBPC3 as referred to herein a wide range of immunoassay techniques using such an assay format are available, see, e.g., U.S. Pat. Nos. 4,016,043, 4,424,279, and 4,018,653. These include both single-site and two-site or "sandwich" assays of the non-competitive types, as well as in the traditional competitive binding assays. These assays also include direct binding of a labeled antibody to a target biomarker.

Preferably, the detection is based on electrochemiluminescence. Methods employing electrochemiluminescent labels are well-known. Such methods make use of the ability of special metal complexes to achieve, by means of oxidation, an excited state from which they decay to ground state, emitting electrochemiluminescence. For review see Richter, M.M., Chem. Rev. 2004;104: 3003-3036. Typically, the electrochemiluminescence immunoassay is carried out via electrochemiluminescence immunoassay (ECLIA) that functions via the sandwich principle. A first biotinylated monoclonal antibody, or fragment thereof, such as F(ab')2-fragment of the first antibody and a ruthenylated second monoclonal antibody form sandwich immunoassay complexes with MYBPC3 in the sample. The complexes are then bound to solid-phase streptavidin-coated microparticles. These are captured magnetically onto the electrode surface, leading to chemiluminescence emission upon application of voltage to the electrode, which is measured by a photomultiplier.

In an embodiment of the sandwich assay for the determination of MYBPC3, the assay comprises a biotinylated first monoclonal antibody (or fragment thereof) and a ruthenylated second monoclonal antibody (or fragment thereof). The two antibodies form sandwich immunoassay complexes with the MYBPC3 present in the sample.

Thus, it is envisaged that the first monoclonal antibody, or a fragment thereof (such as F(ab')2-fragment) is biotinylated and that the second monoclonal antibody, or a fragment thereof is ruthenylated.

The present invention further relates to the use of the monoclonal antibody, or fragment thereof, of the present invention, in particular of both antibodies (e.g. in form of a sandwich assay in a sample from a subject for the diagnostic assessment. Preferably, the antibodies (or fragments thereof) or the sandwich assay is used for determining the amount of the biomarker. The determined amount then allows for the diagnostic assessment.

In a preferred embodiment, cardiac condition is assessed.

The present invention further pertains relates to the *in vitro* use of the monoclonal antibody, or fragment thereof, of the present invention, or of the kit of the present invention for assessing a cardiac condition.

Similarly, the present invention relates to a method for assessing a cardiac condition in a subject, the method comprising

(a) determining the amount of the biomarker MYBPC3 in a sample of the subject,
(b) comparing the amount of the biomarker to a reference amount for the assessment of cardiac condition; and
(c) assessing a cardiac condition based on the comparison made in step (b).

The determination of the amount of the biomarker is typically carried out by

i) contacting a sample comprising MYBPC3 from the subject, such as a blood, serum or plasma sample, with the first monoclonal antibody, or fragment thereof, and/or the second monoclonal antibody, or fragment thereof, of the invention, thereby forming a complex comprising MYBPC3 and said monoclonal antibody (or both monoclonal antibodies), or fragment (s) thereof, and

ii) determining the amount of the complex formed in step (i), thereby determining the amount of MYBPC3 in said sample.

Typically, the sample is contacted with both antibodies or fragments thereof.

In a preferred embodiment, the cardiac condition to be assessed is heart failure. Preferably, the assessment of cardiac condition the diagnosis of heart failure in a subject suspected to suffer from heart failure.

In another preferred embodiment, the cardiac condition to be assessed is myocardial injury.

In another preferred embodiment, the cardiac condition to be assessed is myocardial infarction.

In a preferred embodiment, the assessment of myocardial infarction is the diagnosis of myocardial infarction. In particular, the assessment of myocardial infarction is the rule-out of myocardial infarction. Typically, the subject to be tested is a subject suspected to suffer from myocardial infarction. Thus, the subject may be a patient presenting with suspected acute coronary syndrome.

In another preferred embodiment, the assessment of myocardial infarction is the differentiation between Type 1 and Type 2 myocardial infarction

In another preferred embodiment, the assessment of myocardial infarction is the prediction of the risk of risk myocardial infarction and/or cardiovascular death days for patients suspected of myocardial infarction. For example, the risk at 30 days (and thus within 30 days) is predicted.

In an embodiment, the kit of the present invention, in particular the sandwich assay can be used in the in the identification of patients at low risk of myocardial infarction in patients presenting with suspected acute coronary syndrome. Typically, the kit is used as an aid in said identification. Accordingly, the kit is used in conjunction of a clinical assessment.

Accordingly, the present invention relates to a method for identifying a patient at low risk of myocardial infarction in a patient presenting with suspected acute coronary syndrome, the method comprising

(a) determining the amount of the biomarker MYBPC3 in a sample of the subject,
(b) comparing the amount of the biomarker to a reference amount for the identification; and
(c) identifying a patient at low risk of myocardial infarction based on the comparison made in step (b).

Typically, an amount of the biomarker below the reference amount is indicative for a patient who is at low risk of myocardial infarction. Also typically, an amount of the biomarker above the reference amount is indicative for a patient who is not at low risk of myocardial infarction.

The determination of the amount of the biomarker is typically carried out is described above. Typically, the sandwich assay is used.

In an alternative embodiment, the kit of the present invention, in particular the sandwich assay can be used as an aid in the identification of patients at low risk of myocardial infarction and major adverse cardiovascular events at 30 days in patients presenting with suspected acute coronary syndrome.

A patient who is at low risk of myocardial infarction, typically, is a patient who can be ruled out to suffer from myocardial infarction. Thus, myocardial infarction can be excluded. Typically, said patient can be discharged from the hospital. Accordingly, the antibodies (or fragments thereof) or the kit (such as the sandwich assay) can be used as an aid to exclude myocardial infarction in patients presenting with suspected acute coronary syndrome. Typically, this is done in conjunction with a clinical assessment.

Accordingly, the present invention relates to a method for excluding myocardial infarction in a patient presenting with suspected acute coronary syndrome, the method comprising

(a) determining the amount of the biomarker MYBPC3 in a sample of the subject,
(b) comparing the amount of the biomarker to a reference amount for the identification; and
(c) excluding myocardial infarction in said patient based on the comparison made in step (b).

Typically, an amount of the biomarker below the reference amount is indicative for excluding myocardial infarction, i.e. for ruling out myocardial infarction. Also typically, an amount of the biomarker above the reference amount indicates that myocardial infarction cannot be ruled out.

In an embodiment, the patient with suspected acute coronary syndrome is a patient presenting to an emergency department with suspected acute coronary syndrome.

The determination of the amount of the biomarker is typically carried out is described above. Typically, the sandwich assay is used.

The term "type 1 myocardial infarction" is well-known in the art. As used herein, the term refers to an AMI with the underlying ischemic etiology of an atherosclerotic plaque rupture/erosion with occlusive or non-occlusive thrombus formation in one or multiple coronary arteries. Criteria for type 1 AMI therefore include previously mentioned criteria of an AMI including, for example, identification of a coronary thrombus by angiography including intracoronary imaging or autopsy as evidence of acute myocardial ischemia (Thygesen et al, Eur J Heart 2019:40(3);237-269, DOI:10.1093/eurheartj/ehy462).

The term "type 2 myocardial infarction" is well-known in the art. As used herein, the term refers to an AMI with the underlying ischemic etiology of an impaired oxygen supply-demand balance due to a) an increase in oxygen demand (e.g. sustained tachyarrhythmias or severe hypertension), or b) due to a decrease in oxygen supply (e.g. hypotension, bradyarrhythmias, respiratory failure, severe anemia, coronary vasospasms, coronary artery dissection or microvascular dysfunction). Accordingly, criteria for a type 2 AMI include previously mentioned criteria of an AMI including evidence of an imbalance between myocardial oxygen supply and demand unrelated to acute coronary athero-thrombosis (Thygesen et al, Eur J Heart 2019:40(3);237-269, DOI:10.1093/eurheartj/ehy462).

In the following, preferred embodiments of the invention are summarized. The definitions and explanations provided herein above, preferably, apply mutatis mutandis.

1. A monoclonal antibody, or fragment thereof, which binds to MYBPC3 (Myosin binding protein C, cardiac type), wherein said monoclonal antibody, or fragment thereof, is selected from

i) a first monoclonal antibody, or fragment thereof, comprising a first light chain variable domain and/or a first heavy chain variable domain,

(a) said first light chain variable domain comprising

(a1) a first light chain CDR1 that differs by not more than a total of three amino acid additions, substitutions, and/or deletions from said first light chain CDR1 having an amino acid sequence as shown in SEQ ID NO: 1 (QASQSIGNALA),

(a2) a first light chain CDR2 that differs by not more than a total of three amino acid additions, substitutions, and/or deletions from said first light chain CDR2 having an amino acid sequence as shown in SEQ ID NO: 2 (STSYLPS), and/or

(a3) a first light chain CDR3 that differs by not more than a total of three amino acid additions, substitutions, and/or deletions from said first light chain CDR3 having an amino acid sequence as shown in SEQ ID NO: 3 (QSYYVSTSSSDRSS),

(b) said first heavy chain variable domain comprising

(b1) a first heavy chain CDR1 that differs by not more than a total of three amino acid additions, substitutions, and/or deletions from said first heavy chain CDR1 having an amino acid sequence as shown in SEQ ID NO: 4 (SGYDMC),

(b2) a first heavy chain CDR2 that differs by not more than a total of three amino acid additions, substitutions, and/or deletions from said first heavy chain CDR2 having an amino acid sequence as shown in SEQ ID NO: 5 (CIGSSSASTWYANWVNG), and/or

(b3) a first heavy chain CDR3 that differs by not more than a total of three amino acid additions, substitutions, and/or deletions from said first heavy chain CDR3 having an amino acid sequence as shown in SEQ ID NO: 6 (ESSTEYYYNL),

or

ii) a second monoclonal antibody, or fragment thereof, comprising a second light chain variable domain and/or a second heavy chain variable domain,

(a) said second light chain variable domain comprising

(a1) a second light chain CDR1 that differs by not more than a total of three amino acid additions, substitutions, and/or deletions from a second light chain CDR1 having an amino acid sequence as shown in SEQ ID NO: 9 (RASQNIGTNMH),

(a2) a second light chain CDR2 that differs by not more than a total of three amino acid additions, substitutions, and/or deletions from a second light chain CDR2 having an amino acid sequence as shown in SEQ ID NO: 10 (FASESFS), and/or
(a3) a second light chain CDR3 that differs by not more than a total of three amino acid additions, substitutions, and/or deletions from a second light chain CDR3 having an amino acid sequence as shown in SEQ ID NO: 11 (QQSYNWPFT),

(b) said second heavy chain variable domain comprising

(b1) a second heavy chain CDR1 that differs by not more than a total of three amino acid additions, substitutions, and/or deletions from said second heavy chain CDR1 having an amino acid sequence as shown in SEQ ID NO: 12 (GYGVN),
(b2) a second heavy chain CDR2 that differs by not more than a total of three amino acid additions, substitutions, and/or deletions from said second heavy chain CDR2 having an amino acid sequence as shown in SEQ ID NO: 13 (MIWGDGSTDYNSVYKS), and/or
(b3) a second heavy chain CDR3 that differs by not more than a total of three amino acid additions, substitutions, and/or deletions from said second heavy chain CDR3 having an amino acid sequence as shown in SEQ ID NO: 14 (RDTGGASMDY).

2. The monoclonal antibody or fragment thereof of embodiment 1, wherein

i) said first light chain variable domain is at least 85%, such as at least 90% or at least 95% identical to the light chain variable domain having a sequence as shown in SEQ ID NO: 7 (DIVMTQTPASVEAAVGGTVTIKC QASQSIGNALA WYQQKPGQPPKLLIY STSYLPS GVPSRFKGGGSGAEYTLTISDLECADAATYYC QSYYVSTSSSDRSS FGGGTEVVVK), and wherein the first heavy chain variable domain is at least 85%, such as at least 90% or at least 95% identical to the heavy chain variable domain having a sequence as shown in SEQ ID NO: 8

(QEQLEESGGGLVKPGGTLTLTCKVSGFDFS SGYDMC WVRQAPGKGLESIA CIGSSSASTWYANWVNG RFTVSRSTSLNTVDLKMTSLTVADTATYFCAR ESSTEYYYNL WGPGTLVTVSS),

or
ii) said second light chain variable domain is at least 85%, such as at least 90% or at least 95% identical to the light chain variable domain having a sequence as shown in SEQ ID NO: 15 (DILMTQSPAILSVSPGERVSFSC RASQNIGTNMH WYQQRTNGSPRLLIR FASESFS GIPSRFSGTGSGTDFTLRINSVESEDIADYYC QQSYNWPFT FGAGTKLELK), and wherein the second heavy chain variable domain is at least 85%, such as at least 90% or at least 95% identical to the heavy chain variable domain having a sequence as shown in SEQ ID NO: 16 (QVQLKESGPGLVAPSQSLSITCTVSGFSLT GYGVN WVRQPPGKGLEWLG MIWGDGSTDYNSVYKS

RLSISKDNSKSQVFLKMNSLQTDDTARYYCAR RDTGGASMDY WGQGTSVTVSS).

3. The monoclonal antibody or fragment of embodiment 1 or 2, wherein

i) the first light chain variable domain comprises a sequence as shown in SEQ ID NO: 7, and the first heavy chain variable domain comprises a sequence as shown in SEQ ID NO: 8,
ii) the second light chain variable domain comprises a sequence as shown in SEQ ID NO: 15, and the second heavy chain variable domain comprises a sequence as shown in SEQ ID NO: 16.

4. The monoclonal antibody, or fragment thereof, of any one of embodiments 1 to 3, wherein

i) the first monoclonal antibody, or fragment thereof, comprises

(a) a first light chain variable domain comprising

(a1) a first light chain CDR1 that differs by not more than one amino acid addition, substitution or deletion from said first light chain CDR1 having an amino acid sequence as shown in SEQ ID NO: 4 (QASQSIGNALA),

(a2) a first light chain CDR2 that differs by not more than one amino acid addition, substitution or deletion from said first light chain CDR2 having an amino acid sequence as shown in SEQ ID NO: 5 (STSYLPS), and/or

(a3) a first light chain CDR3 that differs by not more than one amino acid addition, substitution or deletion from said first light chain CDR3 having an amino acid sequence as shown in SEQ ID NO: 6 (QSYYVSTSSSDRSS),

(b) a first heavy chain variable domain comprising

(b1) a first heavy chain CDR1 that differs by not more than one amino acid addition, substitution or deletion from a first heavy chain CDR1 having an amino acid sequence as shown in SEQ ID NO: 7 (SGYDMC),

(b2) a first heavy chain CDR2 that differs by not more than one amino acid addition, substitution or deletion from a first heavy chain CDR2 having an amino acid sequence as shown in SEQ ID NO: 8 (CIGSSSASTWYANWVNG), and/or

(b3) a first heavy chain CDR3 that differs by not more than one amino acid addition, substitution or deletion from a first heavy chain CDR3 having an amino acid sequence as shown in SEQ ID NO: 9 (ESSTEYYYNL),

ii) the second monoclonal antibody, or fragment thereof, comprises

(a) a second light chain variable domain comprising

(a1) a second light chain CDR1 that differs by not more than one amino acid addition, substitution or deletion from a second light chain CDR1 having an amino acid sequence as shown in SEQ ID NO: 9 (RASQNIGTNMH),

(a2) a second light chain CDR2 that differs by not more than one amino acid addition, substitution or deletion from a second light chain CDR2 having an amino acid sequence as shown in SEQ ID NO: 10 (FASESFS), and/or

(a3) a second light chain CDR3 that differs by not more than one amino acid addition, substitution or deletion from a second light chain CDR3 having an amino acid sequence as shown in SEQ ID NO: 11 (QQSYNWPFT),

(b) a second heavy chain variable domain comprising

(b1) a second heavy chain CDR1 that differs by not more than one amino acid addition, substitution or deletion from a second heavy chain CDR1 having an amino acid sequence as shown in SEQ ID NO: 12 (GYGVN),

(b2) a second heavy chain CDR2 that differs by not more than one amino acid addition, substitution or deletion from a second heavy chain CDR2 having an amino acid sequence as shown in SEQ ID NO: 13 (MIWGDGSTDYNSVYKS), and/or

(b3) a second heavy chain CDR3 that differs by not more than one amino acid addition, substitution or deletion from a second heavy chain CDR3 having an amino acid sequence as shown in SEQ ID NO: 14 (RDTGGASMDY).

5. The monoclonal antibody, or fragment thereof, of any one of embodiments 1 to 4, wherein

i) the first monoclonal antibody, or fragment thereof, comprises

(a) a first light chain variable domain comprising

(a1) a first light chain CDR1 having a sequence as shown in SEQ ID NO: 1 (QASQSIGNALA),

(a2) a first light chain CDR2 having a sequence as shown in SEQ ID NO: 2 (STSYLPS), and

(a3) a first light chain CDR3 having a sequence as shown in SEQ ID NO: 3 (QSYYVSTSSSDRSS),

and
(b) a first heavy chain variable domain comprising

(b1) a first heavy chain CDR1 having a sequence as shown in SEQ ID NO: 4 (SGYDMC),
(b2) a first heavy chain CDR2 having a sequence as shown in SEQ ID NO: 5 (CIGSSSASTWYANWVNG), and
(b3) a first heavy chain CDR3 having a sequence as shown in SEQ ID NO: 6 (ESSTEYYYNL),

ii) the second monoclonal antibody, or fragment thereof, comprises

(a) a second light chain variable domain comprising

(a1) a second light chain CDR1 having a sequence as shown in SEQ ID NO: 9 (RASQNIGTNMH),
(a2) a second light chain CDR2 having a sequence as shown in SEQ ID NO: 10 (FASESFS), and
(a3) a second light chain CDR3 having a sequence as shown in SEQ ID NO: 11 (QQSYNWPFT),

and
(b) a second heavy chain variable domain comprising

(b1) a second heavy chain CDR1 having a sequence as shown in SEQ ID NO: 12 (GYGVN),
(b2) a second chain CDR2 having a sequence as shown in SEQ ID NO: 13 (MIWGDGSTDYNSVYKS), and
(b3) a second heavy chain CDR3 having a sequence as shown in SEQ ID NO: 14 (RDTGGASMDY).

6. The monoclonal antibody, or fragment thereof, of any one of embodiments 1 to 5, wherein the first antibody, or fragment thereof, and/or the second antibody, or fragment thereof, is (are) labelled, and/or
wherein the fragment is a Fab fragment, a Fab' fragment, a Facb fragment, a F(ab')2 fragment, a scFv fragment, or a Fv fragment, in particular wherein the fragment is a F(ab')2 fragment.

7. A kit comprising i) the first antibody, or fragment thereof, as defined in any one of the preceding embodiments, and ii) the second antibody, or fragment thereof, as defined in any one of the preceding embodiments.

8. The kit of embodiment 7, wherein the kit comprises a biotinylated F(ab')2 fragment of the first monoclonal antibody and the second monoclonal antibody, preferably wherein the second antibody is ruthenylated.

9. *In vitro* use of the monoclonal antibody, or fragment thereof, of any one of embodiments 1 to 6, or of the kit of embodiment 7 or 8 for the detection of MYBPC3 in a sample.

10. The *in vitro* use of embodiment 9, wherein the detection is the quantitative detection.

11. The *in vitro* use of embodiment 9 or 10, wherein the sample is a body fluid, such as a blood, serum or plasma sample.

12. A method for detecting MYBPC3 in a sample, comprising

(a) contacting a sample comprising MYBPC3 with the first and/or second monoclonal antibody, or fragment thereof, of any one of embodiments 1 to 6, thereby forming a complex comprising MYBPC3 and said monoclonal antibody, or fragment thereof, and
(b) detecting the complex formed in step (a), thereby detecting MYBPC3 in said sample.

13. A complex comprising

i) MYBPC3, and
ii) the first monoclonal antibody, or fragment thereof, and/or the second monoclonal antibody, or fragment thereof, of any one of embodiments 1 to 6.

14. *In vitro* use of the monoclonal antibody, or fragment thereof, of any one of embodiments 1 to 6, or of the kit of embodiment 7 or 8 for assessing a cardiac condition.

15. The *in vitro* use of embodiment 14, wherein the cardiac condition is heart failure.

16. The *in vitro* use of embodiment 14, wherein the cardiac condition is myocardial injury or myocardial infarction.

17. The *in vitro* use of embodiment 16, wherein the cardiac condition is myocardial infarction.

18. The *in vitro* use of embodiment 17, wherein myocardial infarction is ruled out.

19. The *in vitro* use of embodiment 17, wherein it is differentiated between Type 1 and Type 2 myocardial infarction.

20. The *in vitro* use of embodiment 17, wherein the risk of myocardial infarction and/or cardiovascular death for patients suspected of acute coronary syndrome (ACS) is predicted, for example the risk at 30 days.

21. A kit comprising

    i. a first monoclonal antibody, or antigen-binding fragment thereof, which binds to MYBPC3 (Myosin binding protein C, cardiac type), wherein said first monoclonal antibody, or antigen-binding fragment thereof comprises a first light chain variable domain and a first heavy chain variable domain, wherein

        a) the first light chain variable domain comprises a sequence as shown in SEQ ID NO: 7 (DIVMTQTPASVEAAVGGTVTIKC QASQSIGNALA WYQQKPGQPPKLLIY STSYLPS GVPSRFKGGGSGAEYTLTISDLECADAATYYC QSYYVSTSSSDRSS FGGGTEVVVK),

and
b) the first heavy chain variable domain comprises a sequence as shown in SEQ ID NO: 8 (QEQLEESGGGLVKPGGTLTLTCKVSGFDFS SGYDMC WVRQAPGKGLESIA CIGSSSASTWYANWVNG RFTVSRSTSLNTVDLKMTSLTVADTATYFCAR ESSTEYYYNL WGPGTLVTVSS)

and
ii. a second monoclonal antibody, or antigen-binding fragment thereof, which binds to MYBPC3 (Myosin binding protein C, cardiac type), wherein said second monoclonal antibody, or antigen-binding fragment thereof comprises a second light chain variable domain and a second heavy chain variable domain, wherein

        a) the second light chain variable domain comprises a sequence as shown in SEQ ID NO: 15 (DILMTQ-SPAILSVSPGERVSFSC RASQNIGTNMH WYQQRTNGSPRLLIR FASESFS GIPSRFSGTGSGTDFTL-RINSVESEDIADYYC QQSYNWPFT FGAGTKLELK), and
b) the second heavy chain variable domain comprises a sequence as shown in SEQ ID NO: 16 (QVQLKESGPGLVAPSQSLSITCTVSGFSLT GYGVN WVRQPPGKGLEWLG MIWGDGSTDYNSVYKS RLSISKDNSKSQVFLKMNSLQTDDTARYYCAR RDTGGASMDY WGQGTSVTVSS).

22. The kit of embodiment 21, wherein the kit comprises i) a biotinylated F(ab')2 fragment of the first monoclonal antibody and ii) a ruthenylated second monoclonal antibody.

23. In vitro use of the antibody or the kit of any one of the preceding embodiments as an aid in the identification of patients at low risk of myocardial infarction in patients presenting with suspected acute coronary syndrome.

24. In vitro use of the antibody or the kit of any one of the preceding embodiments for the *in vitro* quantitative determination of cardiac myosin binding protein C (cMyBP-C) in human serum and plasma.

25. A method for assessing a cardiac condition in a subject, the method comprising

    (a) determining the amount of the biomarker MYBPC3 in a sample of the subject,
    (b) comparing the amount of the biomarker to a reference amount for the assessment of cardiac condition; and
    (c) assessing a cardiac condition based on the comparison made in step (b).

26. A method for identifying a patient at low risk of myocardial infarction, the method comprising

    (a) determining the amount of the biomarker MYBPC3 in a sample from a patient presenting with suspected acute coronary syndrome,
    (b) comparing the amount of the biomarker to a reference amount for the identification; and
    (c) identifying a patient at low risk of myocardial infarction based on the comparison made in step (b).

27. A method for aiding in the exclusion of myocardial infarction in a patient presenting with suspected acute coronary syndrome, the method comprising

    (a) determining the amount of the biomarker MYBPC3 in a sample of the subject,
    (b) comparing the amount of the biomarker to a reference amount for the identification; and
    (c) aiding in the exclusion of myocardial infarction in said patient based on the comparison made in step (b).

28. The method of any one of embodiments 25 to 27, wherein the determination in step a) comprises

    i) contacting a sample comprising MYBPC3 from the subject, such as a human blood, serum or plasma sample, with the first monoclonal antibody, or fragment thereof, and/or the second monoclonal antibody, or fragment thereof, of the invention, thereby forming a complex comprising MYBPC3 and said monoclonal antibody (or both monoclonal antibodies), or fragment (s) thereof, and
    ii) determining the amount of the complex formed in step (i), thereby determining the amount of MYBPC3 in said sample.

29. *In vitro* use of the antibody (or fragment thereof) or the kit of any one of the preceding embodiments in human, blood, serum or plasma samples as an aid to exclude myocardial infarction in patients presenting with suspected acute coronary syndrome.

30. The method of embodiment 27 or 28, or the in vitro use of embodiment 29, wherein the patient with suspected acute coronary syndrome is a patient presenting to an emergency department with suspected acute coronary syndrome.

All patents, patent applications, and publications or public disclosures referred to or cited herein are incorporated by reference in their entirety.

The invention will be further described with reference to the examples described herein; however, it is to be understood that the invention is not limited to such examples.

**Example 1: Immunogen**

Recombinant expression of MYBPC3

Full-length human cardiac-type Myosin-binding protein C3, Uniprot Q14896, is a large and difficult-to-express protein (data not shown).

In contrast, the N-terminal residues 1 to 330 of Uniprot Q14896, fused to a C-terminal His-Tag for purification resulted in a soluble and reproducible expression in human embryonic kidney (HEK) 293 cells. The 330 N-terminal residues including the C-terminal His-Tag are shown in SEQ ID NO: 17:

```
  1  MPEPGKKPVS AFSKKPRSVE VAAGSPAVFE AETERAGVKV RWQRGGSDIS
 51  ASNKYGLATE GTRHTLTVRE VGPADQGSYA VIAGSSKVKF DLKVIEAEKA
101  EPMLAPAPAP AEATGAPGEA PAPAAELGES APSPKGSSSA ALNGPTPGAP
151  DDPIGLFVMR PQDGEVTVGG SITFSARVAG ASLLKPPVVK WFKGKWVDLS
201  SKVGQHLQLH DSYDRASKVY LFELHITDAQ PAFTGSYRCE VSTKDKFDCS
251  NFNLTVHEAM GTGDLDLLSA FRRTSLAGGG RRISDSHEDT GILDFSSLLK
301  KRDSFRTPRD SKLEAPAEED VWEILRQAPP GGGSHHHHHH HH  (SEQ ID 17)
```

SEQ ID NO: 18 shows the amino acid sequence without the His-Tag:

```
  1   MPEPGKKPVS AFSKKPRSVE VAAGSPAVFE AETERAGVKV RWQRGGSDIS
 51   ASNKYGLATE GTRHTLTVRE VGPADQGSYA VIAGSSKVKF DLKVIEAEKA
101   EPMLAPAPAP AEATGAPGEA PAPAAELGES APSPKGSSSA ALNGPTPGAP
151   DDPIGLFVMR PQDGEVTVGG SITFSARVAG ASLLKPPVVK WFKGKWVDLS
201   SKVGQHLQLH DSYDRASKVY LFELHITDAQ PAFTGSYRCE VSTKDKFDCS
251   NFNLTVHEAM GTGDLDLLSA FRRTSLAGGG RRISDSHEDT GILDFSSLLK
301   KRDSFRTPRD SKLEAPAEED VWEILRQAPP (SEQ ID NO: 18)
```

All expression plasmids featured a human cytomegalovirus immediate-early enhancer/promoter region, and the coding gene region was followed by a bovine growth hormone polyadenylation signal.

For transient gene expression, the FreeStyle™ 293-F expression system (Thermo Fischer Scientific) was used: Cultures at ~ 2 × 106 cells/ml were transfected with the expression plasmid at a concentration of 0.5 mg/l cell culture complexed by the PEIpro™ transfection reagent (Polyplus) according to the manufacturer's guidelines. 72 hrs post-transfection, the cell pellet was collected by centrifugation and stored at -80°C for cell lysis and subsequent protein purification

Purification and Characterization of MYBPC3

The frozen cells were thawed, resuspended in 50 mM HEPES pH 7.5, 300 mM NaCl, 5 mM MgCl2, 5% glycerol, 0.1% tween 20. Protease inhibitor (10 tablets Complete EDTA free (Roche) per 100 ml) and DNAse I were added to the buffer directly before use. A Potter-Elvehjem Homogenizer was used for cell lysis.

For purification, the clarified lysate was applied on an IMC chromatography. The His-tagged myBC3 was eluted with imidazole and dialyzed into the eluting buffer of the following 50 mM HEPES pH 7.5, 150 mM NaCl, 6.5% Sucrose. The monomeric MYBPC3 was obtained by the final polishing step on a preparative size exclusion chromatography with a Superose 200 increase column.

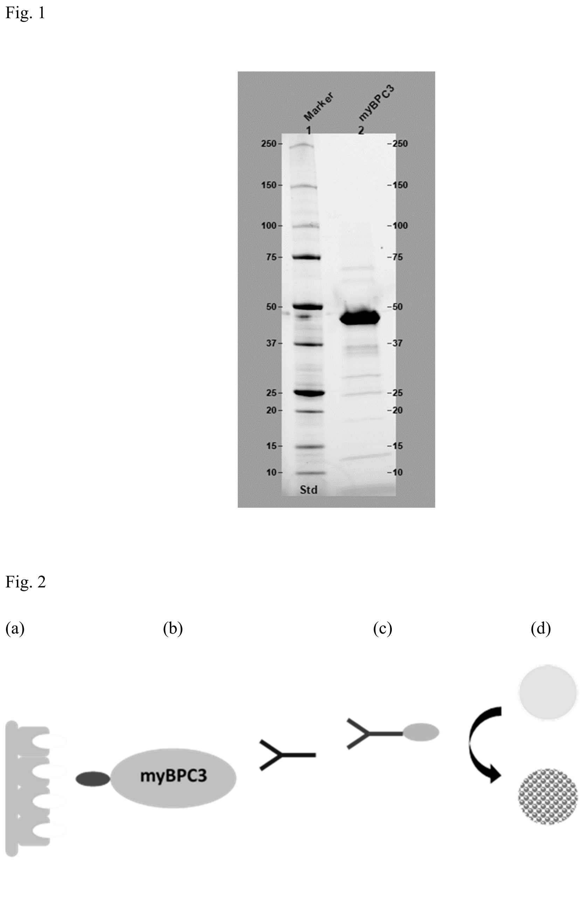

The purified MYBPC3 was characterized by SDS Page (a) and SEC-RALS HPLC (b) as a monomeric, glycosylated protein with a purity of > 85%. The results are shown in Figure 1.

**Example 2: Immunization and Antibody screening by ELISA**

Rabbit immunization (B-cell-antibodies)

In order to generate antibodies against structural parts of MYBPC3, rabbits were immunized with recombinant purified immunogen (MYBPC3-Avi-His; see above). After 42 days of immunization, blood samples (30 mL) were collected from the immunized rabbits. The blood samples were purified and B-cells were isolated by a single-cell-sorting (FACS). The isolated cells were inoculated by the use of feeder cells. Supernatants were tested on their reactivity against native recombinant MYBPC3 with an ELISA-assay and further analyzed by an initial kinetic screening using Surface Plasmon Resonance spectroscopy (SPR), known as Biacore (next section).

The ELISA-Screening was performed with supernatants from B-cell-cultivations in the following 96- or 384-microtitre-assay-format, respectively (see Figure 2).

Positive, MYBPC3-specific antibody producing B-cells were subcloned in suitable expression vectors (*E. coli*) and expressed recombinantly (heavy chain and light chain) in HEK293-cells. Supernatants of suitable clones were transferred for further investigation.

The following clones were transferred: 4H4, 4H7, 4H11, 5B8, 6C6, 6G5, 7B5, 7D5, 8H1, 11F2, 13F11, 14G6, 25G6, 25H1.

Mouse immunization (hybridoma)

To generate mouse antibodies against structural parts of myBP3, Balb/c- and NMRI-mouse strains were immunized with recombinant purified immunogen (MYBPC3-Avi-His; see above). After immunization, spleens of the immunized animals were prepared and fused to myeloma-cells (Ag8). The resulting hybridoma-cells were cultivated and supernatants were tested by ELISA (see Fig. 2) and SPR-based kinetic screening described below) for their reactivity against rec MYBPC3. Positive primary cultures were utilized to generate single clones. After cultivation, supernatants were tested again for their reactivity against MYBPC3. Positive clones were then cultivated in larger amounts (CELLine) and transferred for further investigations.

The following supernatants containing monoclonal antibodies were transferred: clones 4.1.1, 4.2.1, 4.6.6 and 4.7.10.

**Example 3: initial kinetic screening by SPR**

Surface Plasmon Resonance (SPR)-Spectroscopy - assisted kinetic screening and detailed characterization were performed using GE Healthcare Biacore™ 3000, T200 or 8K + instruments.

Functionalizing the Sensor - Amine coupling capture systems:

A rabbit resp. a mouse antibody capture system was immobilized on a CM5 sensor surface. A polyclonal goat anti-rabbit IgG Fc capture antibody GARbFcγ (Code-No. 111-005-046; Jackson Immuno Research) resp. a polyclonal rabbit anti-mouse IgG Fc capture antibody pAb<M-IgG>Rb-IgG, (GE Healthcare BR-1008-38) or polyclonal rabbit anti-mouse IgG Fc RAMFcγ (Code-No. 315-005-046, Jackson Immuno Research) was amine coupled using the EDC/NHS-chemistry according to the manufacturer's instructions with densities of 9000-15000 RU

Kinetic characterization for antibody-interactions:

All MYBPC3 interactions were analyzed at 37°C. The system buffer was HBS-ET pH 7.4, 10 mM HEPES, 150 mM NaCl, 3 mM EDTA, 0.05% (w/v) Tween20. The system buffer was supplemented with 1 mg/mL CMD (Carboxymethyl-Dextran, Sigma, Cat. No. 86524) and was used as a sample buffer for the preparation of dilution series.

When using the 8K instruments, flow cells (Fc)1 of channels 1-8 served as references and Fc2 of channels 1-8 were used for the interaction measurements. When using the B3000- or T200 instrument, Fc1 served as reference and Fc2, 3 and 4 were used for the interaction measurements.

The anti-MYBPC3 antibody supernatants were diluted 1:3 - 1:5 (mouse) resp. 1: 100 (rabbit) in the sample buffer and were injected for 2 minutes.

The antibody Capture Level (CL) in resonance units RU was monitored. Next, 150 nM MYBPC3 (Roche in house, 38.7 kDa) for the kinetic screening was injected at 30 or 60 μl/min to the surface displayed anti-MYBPC3 Antibodies.

The analyte association phase was monitored for 3 minutes and the dissociation phase was monitored for 5 minutes (kinetic screening). After each measurement cycle, the capture systems were regenerated by subsequent injections of 10 mM Glycine buffers pH 2.0 and pH 2.25 at 20 μL/min for 60 seconds resp. according to the manufacturer's instructions.

The binding signatures were monitored by the BIAcore™ B3000 Control and Evaluation SW V4.1, the T200 Control SW 2.0.1 and Evaluation SW 3.0 resp. 8K Control-SW V3.0.11.15423 and evaluated by the BIAcore™ Insight Evaluation SW V3.0.11.15423. Furthermore, the kinetic data were interpreted by report point characterizations and kinetic determinations. Two report points, the recorded signal shortly before the end of the analyte injection, Analyte Binding Late (BL), and the signal shortly before the end of the dissociation time, Stability Late (SL), were used to characterize the antibody/antigen binding stability.

The antibody/antigen complex half-life was calculated in minutes according to the formula

$$t/2\ \mathrm{diss} = \ln(2)/(kd*60).$$

The Molar Ratio, the binding stoichiometry was calculated by the formula

$$MR = BL(\mathrm{Antigen})* MW(\mathrm{Antibody})/ (MW(\mathrm{antigen})* CL\ (\mathrm{Antibody})).$$

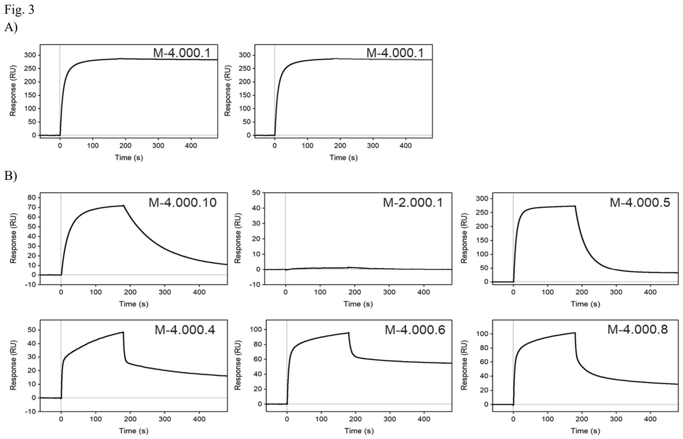

Fig. 3 shows the kinetic profiles for antibodies from mouse supernatants binding MYBPC3 at 37°C.

**Example 4: Assay Development**

Testing on ELISA-MTP platform

The assay is a sandwich-ELISA format with a biotinylated antibody binding to a streptavidin-coated micro titer plate (MTP) and a digoxigenin (DIG)-labeled detection-antibody. Both antibodies are specific to MYBPC3. In a next step a Anti-Digoxigenin antibody labeled with peroxidase binds to the DIG. Then, Tetramethylbenzidine (TMB) is added, the reaction is stopped by adding sulphuric acid. Photometric detection of substrate turnover by applying a photometric MTP reader at 450 nm and at 540 nm as reference.

To test for efficient antibody binding the recombinantly expressed N-terminal MyBPC-Fragment (1-330) (the immunogen as described above) was used as sample.

Table 1: Screening of mouse mAB for best differentiation of 1ng/ml MYBPC3 relative to blank

| Ratio: ($\Delta$ OD 1 ng/mL MYBPC3) / blank | | | |
|---|---|---|---|
| Dig-AK | 4.1.1 | 4.7.10 | 4.6.6 |
| Bi-AK | | | |
| 4.1.1 | 1.09 | 23.56 | 3.39 |
| 4.7.10 | 55.06 | 1.43 | 14.75 |
| 4.6.6 | 13.68 | 6.09 | 1.61 |

Conclusion: while mABs 4.1.1 and 4.7.10 do efficiently form sandwich complexes with MYBPC3, 4.6.6 is less suited and de-selected for the further development.

Next, formation of orthogonal sandwich complexes comprising mouse and rabbit antibodies was tested in the ELISA-MTP-format.

Table 2: Testing orthogonal complex formation between mouse mAbs and rabbit mAbs, screening for best differentiation of 2.7pg/ml MYBPC3 relative to blank.

| Ratio: ($\Delta$ OD 2.7 pg/mL/blank) | | | | |
|---|---|---|---|---|
| Dig-AK | 4.1.1 | 4.7.10 | 7B5 | 5B8 |
| Bi-AK | | | | |
| 4.1.1 | | | 1.43 | 0.99 |
| 4.7.10 | | | 1.01 | 1.01 |
| 7B5 | 3.07 | 0.98 | 0.98 | 1.01 |
| 5B8 | 0.98 | 0.98 | 1.01 | 1.00 |

Conclusion: orthogonal sandwich-complex formation is possible.

The final selection of the best sandwich antibody pair was carried out on the Elecsys platform.

Testing on Elecsys system:

An electrochemiluminescence immunoassay (ECLIA) for the specific measurement of MYBPC3 in particular in human serum or plasma samples was developed using the Elecsys® cobas analyzer e601. The Elecsys MYBPC3 immunoassay is an electrochemiluminescence immunoassay (ECLIA) that functions via the sandwich principle. There are two antibodies included in the assay, a biotinylated monoclonal antibody candidate as capture antibody and a ruthenylated monoclonal antibody candidate as detection antibody, which form sandwich immunoassay complexes with MYBPC3 in the sample (here again immunogen as described before is used as MYBPC3 sample). The complexes are then bound to solid-phase streptavidin-coated microparticles. These are captured magnetically onto the electrode surface, leading to chemiluminescence emission upon application of voltage to the electrode, which is measured by a photomultiplier. Results are determined via an instrument-specific calibration curve determined by series of 5 calibrators with different concentrations of MYBPC3 across the measuring range. Samples are measured by pipetting volumes of 50 ul of the sample, 60 ul of reagent 1 (R1), 60 $\mu$l of reagent 2 (R2) and 30 $\mu$l of magnetic beads. R1 is containing the bioinylated mAB in phosphate reaction buffer and reagent 2 (R2) is containing the ruthenium conjugated mAb in the same reaction buffer.

A high sensitive limit of detection is set as selection criterion for the identification of best-suited monoclonal antibody sandwich pairs.

Table 3: Testing in Elecsys Format - screening of various mouse mAB : rabbit mAB as well as rabbit mAB : rabbit mAB combinations, screening for best differentiation of 0.9 pg/ml MYBPC3 (3a) and 2.7 pg/ml MYBPC3 (3b) relative to blank.

| 3a: ratio (0.9 pg/mL) / blank | | | | | | | | |
|---|---|---|---|---|---|---|---|---|
| Ru-AK | 4.1.1 | 5B8 | 7D5 | 8H1 | 11F2 | 7B5 | 13F11 | 25H1 |
| Bi-AK | | | | | | | | |
| 4.1.1 | - | 0.99 | 1.02 | 1.02 | 1.01 | 1.11 | 1.14 | 1.01 |
| 5B8 | 1.01 | - | 1.01 | 1.00 | 1.02 | - | - | - |
| 7D5 | 1.04 | 0.99 | - | 1.03 | 1.00 | 0.99 | 0.98 | - |
| 8H1 | 1.00 | 0.99 | 0.99 | - | 0.98 | - | - | - |
| 11F2 | 0.98 | 1.00 | 0.99 | 1.00 | - | - | - | - |
| 7B5 | 1.18 | - | - | - | - | - | 1.01 | 1.01 |
| 13F11 | 1.13 | - | - | - | - | - | - | - |
| 25H1 | 1.00 | - | - | - | - | 1.03 | - | - |
| 3b: ratio (2.7 pg/mL) /blank | | | | | | | | |
| Ru-AK | 4.1.1 | 5B8 | 7D5 | 8H1 | 11F2 | 7B5 | 13F11 | 25H1 |
| Bi-AK | | | | | | | | |
| 4.1.1 | - | 1.00 | 1.09 | 1.01 | 1.00 | 1.32 | 1.32 | 1.00 |
| 5B8 | 1.01 | - | 1.01 | 1.00 | 1.02 | - | - | - |
| 7D5 | 1.11 | 0.98 | - | 1.02 | 1.00 | 1.00 | 0.98 | |
| 8H1 | 1.01 | 0.97 | 0.98 | - | 1.00 | - | - | - |
| 11F2 | 0.98 | 1.00 | 0.98 | 1.00 | - | - | - | - |
| 7B5 | 1.40 | - | - | - | - | - | 1.02 | 1.03 |
| 13F11 | 1.45 | - | - | - | - | - | - | - |
| 25H1 | 1.01 | - | - | - | - | 1.05 | - | - |

Conclusion: After testing various combinations, the top three pairs are 7B54.1.1, 13F11/4.1.1 (best performing at 0.9 and 2.7 pg/mL) and 7D4/4.1.1 (slightly less efficient).

Table 4: Head-to Head comparison of top 3 mAB combinations over high-sensitive detection range (0-2000 pg/ml MYBPC3)

| MYBPC3 [pg/mL] | 7D5/4.1.1 | 7B5/4.1.1 | 13F11/4.1.1 |
|---|---|---|---|
| 0 | 671 | 651 | 662 |
| 0.9 | 770 | 786 | 785 |
| 2.7 | 919 | 929 | 946 |
| 8.2 | 1306 | 1379 | 1382 |
| 24.7 | 2398 | 2628 | 2589 |
| 74.1 | 5574 | 6205 | 6219 |
| 222.2 | 15237 | 17222 | 16845 |
| 666.7 | 43576 | 48797 | 48725 |

(continued)

| MYBPC3 [pg/mL] | 7D5/4.1.1 | 7B5/4.1.1 | 13F11/4.1.1 |
|---|---|---|---|
| 2000.0 | 127636 | 147008 | 145929 |
| Conclusion: 7B5/4.1.1 was selected. | | | |

**Example 5: SPR - Detailed characterization of selected antibodies 7B5 / M-4.1.1**

Unless stated otherwise, the further detailed kinetic characterization using SPR was performed as stated under example 3 "Initial kinetic screening via SPR".

The purified antibodies were diluted to 1-5 nM and surface displayed via the capture systems as described. A MYBPC3 concentration series with between 0.4-90 nM, dilution factor 3 was analyzed instead of the single concentration.

The analyte association phase was monitored for 3 minutes and the dissociation phase was monitored for 10 minutes, resp. for 30 minutes for 30 nM concentration measured as duplicate.

For the evaluation, the kinetic rate constants and the dissociation equilibrium constants $K_D$ were determined using a Langmuir 1:1 fitting model according to the BIAcore SW. Secondly, the Langmuir 1:1 fitting Scrubber-SW V2.0c was applied.

Kinetic characterization

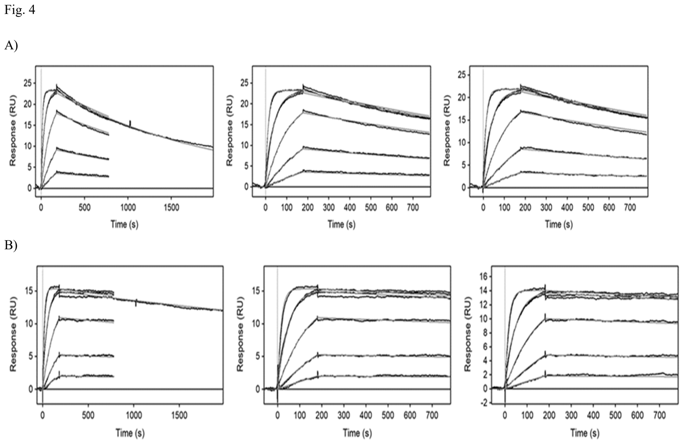

The binding signatures for the detailed characterization of mAbs 7B5 and M-4.1.1 show a fast MYBPC3 complex formation, coming close to the antibody saturation for 30 nM MYBPC3. The 30 minutes dissociation phases show a sufficiently high complex stability for both antibodies. The interactions obeying Langmuir law, see Fig. 4.

Results for the obtained kinetic constants are summarized in Table 5. Both antibodies bind MYBPC3 with high affinities in the picomolar range.

Table 5: Kinetic constants and affinities for clones 7B5 and M-4.1.1

| mAb | $k_a$ [M$^{-1}$s$^{-1}$] | $k_d$ [s$^{-1}$] | $t_{1/2\ diss}$ [min] | $K_D$ **[M]** | Rmax [RU] | CL [RU] | MR [-] |
|---|---|---|---|---|---|---|---|
| 7B5 / 1 | 2.7E+06± 0.1 % | 5.1E-04± 0.1 % | 23 | 1.9E-10±0.1 % | 23.3 | 58 | 1.9 |
| 7B5 / 2 | | | | | 21.9 | 47 | 1.8 |
| M-4.1.1 / 1 | 2.1E+06± 0.2 % | 1.2E-04± 0.2 % | 98 | 5.6E-11±0.2 % | 15.4 | 42 | 1.4 |
| M-4.1.1 / 2 | | | | | 14.1 | 41 | 1.3 |

Sandwich complex formation via SPR

The sandwich complex formation for antibodies M-4.1.1 and 7B5 was performed at 25°C on a GE Healthcare BIAcore™ B3000 instrument.

Biotinylated antibody M-4.1.1-Bi was used as primary, 7B5 was used as secondary antibody, injected to pre-complexed antibody-antigen complex M-4.1.1/ MYBPC3.

A CAP-sensor surface was used according to the manufacturer's instructions. For reversibly capturing, the CAP-reagent is injected for 5 minutes at a flow rate 2 µL/min. Next, the 20 nM M-4.1.1-Bi was injected on Fc2 for 3 minutes at a flow rate 10 µL/min. Fc1 served as reference and was saturated with 50 nM Amino- PEO-Biotin (Sigma Aldrich); 150 nM MYBPC3 resp. buffer as negative control was injected for 3 minutes, flow rate 30 µL/min; Finally, the second antibody 200 nM 7B5, resp. buffer as control was injected for 3 minutes, followed by a 1 minute dissociation. After each cycle, the system was regenerated according to the manufacturer's instructions.

The immune complex stability was monitored and evaluated using the SW from BIAcore™ B3000 Control and Evaluation SW V4.1. The sandwich complex formation experiments were interpreted by visual inspection. The epitope accessibility was quantified as Molar Ratio by forming the ratio between the resonance units of the secondary antibody binding response signal versus the capture level of the primary antibody.

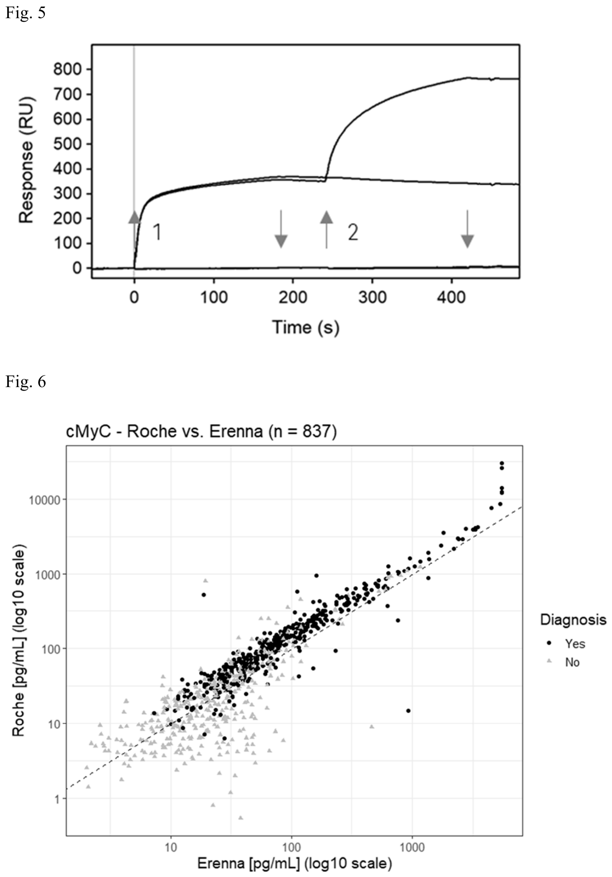

The antibody pair M-4.1.1 and 7B5 forms a sandwich complex with MYBPC3. Both antibodies recognize different epitopes of their target MYBPC3. The sensorgram for the sandwich complex formation with antibody M-4.1.1, surface displayed on the sensor, and 7B5 used as the secondary antibody is shown in figure 5.

The sensorgram of Fig. 5 shows MYBPC3 binding to surface displayed M-4.1.1 with subsequent injection of the 2nd antibody 7B5 forming a sandwich complex. The sensorgram in the middle shows the MYBPC3 injection followed by a buffer injection as negative control instead of the 2nd antibody. The 2 sensorgrams at the bottom shows the negative controls with buffer injected instead of the MYBPC3 and 2nd antibody, respectively buffer instead of MYBPC3 and 2nd antibody 7B5

**Example 6: Epitope Characterization**

Peptide mapping linear peptides

The epitope mapping of antibody clones was carried out by means of a library of immobilized peptides (length: 15 amino acids) corresponding to the canonical sequence of human MYBPC3 (1-392). Cysteine residues were replaced by serine. Peptides were synthesized with an automated synthesizer (Intavis MultiPep RS) on modified cellulose discs which were dissolved after synthesis. The solutions of the individual peptides were then spotted onto coated microscope slides. The synthesis was carried out stepwise utilizing 9-fluorenylmethoxycarbonyl (Fmoc) chemistry on amino-modified cellulose disks in a 384-well synthesis plate. In each coupling cycle, the corresponding amino acids were activated with a solution of DIC/HOBt in DMF. Between coupling steps, unreacted amino groups were capped with a mixture of acetic anhydride, diisopropylethyl amine and 1-hydroxybenzotriazole. Upon completion of the synthesis, the cellulose discs were transferred to a 96-well plate and treated with a mixture of trifluoroacetic acid (TFA), dichloromethane, triisoproylsilane (TIS) and water for side chain deprotection. After removal of the cleavage solution, the cellulose bound peptides were dissolved with a mixture of TFA, TFMSA, TIS and water, precipitated with diisopropyl ether and re-suspended in DMSO. These peptide solutions were subsequently spotted onto Intavis CelluSpots™ slides using an Intavis slide spotting robot.

For epitope analysis, the prepared slides were washed with ethanol and then Tris-buffered saline (TBS; 50 mM Tris, 137 mM NaCl, 2.7 mM KCl, pH 8) before a blocking step was carried out for 1 h at 37 °C with 5 mL 10x Western Blocking Reagent (Roche Applied Science), 2.5 g sucrose in TBS, 0.1% Tween 20. After washing (TBS + 0.1% Tween 20), the slides were incubated with a solution (1 μg/mL) of antibody clones in TBS + 0.1% Tween 20 at 37 °C 1 h. After washing, the slides were incubated for detection with anti-mouse or anti-rabbit secondary HRP-antibodies (1:20000 in TBS-T) followed by incubation with DAB substrate. Positive SPOTs were assigned to the corresponding peptide sequences.

Table 6

| Clone | Epitope |
|---|---|
| MAK<MYPBC3<rK-7B5-IgG(SPA) | no binding |
| MAK<MYPBC3<rK/M-7B5(p11g/fr)-IgG(SPA) | no binding |
| MAK<MYPBC3<M-4.1.1-IgG(SPA) | no binding |
| MAK<MYPBC3<rH-4.1.1(p11ny/gx)-IgGP8(SPA) | no binding |
| MAK<MYPBC3<M-4.6.6-IgG(SPA) | W-E-I-L-R-Q |
| MAK<MYPBC3<M-4.7.10-IgG(SPA) | L-D-F-S-S-L |
| MAK<MYBPC3>rK-11F2-IgGBi(DDS,Sux) | no binding |
| MAK<MYBPC3>rK-7D5-IgGBi(DDS,Sux) | no binding |
| MAK<MYBPC3>rK-8H1-IgGBi(DDS,Sux) | no binding |
| MAK<MYBPC3>rK-5B8-IgG | no binding |
| MAK<MYBPC3>rRb-25H1-IgG | no binding |
| MAK<MYBPC3>rK-13F11-(HC1/LC4)-IgG(SPA) | no binding |
| MAK<MYBPC3>M-4.2.1-IgG(SPA) | no binding |
| *MAK<MYBPC3>M-G7-IgGBi(DDS) | no binding |
| *MAK<MYBPC3>M-G1-IgGBi(DDS) | T-E-R-A-G-V-K-V-R-W |
| *MAK<MYBPC3>M-E7-IgGBi(DDS) | no binding |
| *MAK<MYBPC3>M-F1-IgGBi(DDS) | T-E-R-A-G-V-K-V-R-W |
| *PAK<MYBPC3>S-AF7439-IgGBi(DDS) | no binding |
| * commercially available antibodies against MYBPC3: G7, G1, E7 and F1 available at Santa Cruz, AF7439 available at R&D Systems | |

Conclusion: The epitopes of the selected clones 7B5 and M-4.1.1 do not overlap with the epitopes revealed by King's college in WO2015110114 and WO215110115, nor do they overlap with the epitopes of commercially available clones M-G1 and M-F1. The epitopes of the selected clones 7B5 and M-4.1.1 are probably not linear.

HD-X: Hydrogen/Deuterium Exchange Mass Spectrometry

Antigen: MYBPC3
Antibody 1: 7B5
Antibody 2: M.4.1.1

EXPERIMENTAL

Hydrogen/Deuterium Exchange Mass Spectrometry

MYBPC3 antigen was labeled with deuterium by incubation in buffered, deuterated water (D2O)

a) in the absence of an antibody (protein state 1)
b) in the presence of antibody 7B5 (protein state 2)
c) in the presence of antibody M.4.1.1 (protein state 3)

for various times. After the various labeling times the exchange reaction was quenched by addition of quenching buffer.
Subsequently, all samples were protease digested with Nepenthesin-2, the resulting peptides were separated by reversed-phase UPLC and identified by MS/MS detection using a Thermo Orbitrap Fusion Lumos mass spectrometer.
For data evaluation to identify peptides with different deuteration level in the MYBPC3 antigen in protein state 1, 2 and 3 respectively, the commercially available software tools Peaks Studio from Bioinformatics Solutions Inc. (BSI) and HDExaminer from Sierra Analytics were used.
Details of the experiment are:

A) Tools, reagents and solutions:

Antigen:
MYBPC3, c = 0.5 mg/ml in 50mM HEPES 150mM NaCl, 6,5% Saccharose, pH 7,5

Antibody 1:
7B5, c = 2.1 mg/ml in 50 mM KPP, 150 mM KCl, pH 7,5

Antibody 2:
M.4.1.1 c = 28.4 mg/ml in 50 mM KPP, 150 mM KCl, pH 7,5

H2O-buffer:
50 mM Potassium Phosphate buffer, 150 mM KCl, pH 7,5 in H2O

D2O-buffer:
50 mM Potassium Phosphate buffer, 150 mM KCl, pH 7,5 in D2O.

Quench-buffer:
4 M Urea, 0.17M KH2PO4, 0.3 M TCEP, pH 2.8

Pipetting and labeling robot:
Leap technologies, PAL HTC autosampler

Protein state 1 (antigen alone):
72.38 $\mu$l of the antigen was diluted with 44.62 $\mu$l H2O-buffer and provided in a 0°C tempered sample rack of the robot.

Protein state 2 (antigen plus 7B5):

72.38 µl of the antigen were added to 37.44 µl 7B5 and diluted in 7.18 µl H2O-buffer, also provided in the 0°C tempered sample rack.

Protein state 3 (antigen plus M.4.1.1):
72.38 µl of the antigen were added to 7.02 µl M.4.1.1 and diluted in 37.6 µl H2O-buffer, also provided in the 0°C tempered sample rack.

Labeling times:

0 min
15 s
1, 10, 60, 300 min

B) Labeling of unbound (protein state 1) and bound (protein states 2 and 3) MYBPC3 with deuterium

B1) Peptide identification and 0 min labelling time with subsequent quenching (no deuteration control):
The robot diluted 5.2 µl of protein state 1 into 46.8 µl H2O-buffer in a sample rack at 20 °C. 39 µl thereof were transferred and mixed into 39 µl quench-buffer in the 0°C tempered sample rack. After 20 s quench time 50 µl of this sample were injected into the measurement system.

B2) Various labeling times (15 s, 1, 10, 60 and 300 min) with subsequent quenching:

The robot diluted 5.2 µl of protein state 1 into 46.8 µl D2O-buffer in a sample rack at 20 °C. After the respective labelling time 39 µl thereof were transferred and mixed into 39 µl quench-buffer in the 0°C tempered sample rack. After 20 s quench time 50 µl of this sample were injected into the measurement system.
The same procedure was performed with protein state 2 and 3.
All labelling samples (0 min, 15 s, 1, 10, 60 and 300 min labeling) were performed in triplicates.

C) Analysis of deuterium labeled samples:

Samples from B) were loaded onto a cooled (0°C) Waters NanoAcquity UPLC system (Waters Corp., Milford, MA). Online Nepenthesin-2 digestion (Affipro, # AP-PC-004) was performed at 20°C. The achieved peptides were trapped and desalted at 0°C for 3 min (0.23 % formic acid in water, 200 µl/min, Waters VanGuard C18, 1.7 µm, 2.1x5 mm). Subsequently a reversed phase separation (Waters BEH C18, 1.7 µm, 1x100 mm) using a gradient from 8-35% with 0.23% formic acid in acetonitrile (pH 2.5) in 7 min for all samples, at a flow rate of 40 µl/min was performed.

Mass analysis was performed by a Thermo Orbitrap Fusion Lumos mass spectrometer using positive ion-electrospray.

For peptide identification using the 0min-labeling-time sample (B1) higher-energy collisional dissociation (HCD) was used for the first replicate, electron transfer dissociation for the second replicate and electron-transfer/higher-energy collision dissociation (EThcD) for the third replicate; all in data dependent acquisition (DDA) mode.For all labelling measurements a full scan mode was sufficient.

D) Data evaluation:
Peaks Studio, Bioinformatics Solutions Inc. (BSI) was used for peptide identification according to the instructions of the provider. HDExaminer, Sierra Analytics was used to determine deuterium incorporation in peptides from the different labeling times in comparison to the undeuterated control (0 min labelling time) according to instructions of the provider.

Summary of results:

Table 7

| Epitope regions of antibody 7B5 on MYBPC3 | |
|---|---|
| Epitope regions found on MYBC3 | AA (from - to) |
| -TERAGVKV- (SEQ ID NO: 19) | 33-40 |

(continued)

| Epitope regions of antibody 7B5 on MYBPC3 | |
|---|---|
| -YGLATEGTRHTL- (SEQ ID NO:20) | 55-66 |

Table 8

| Epitope regions of antibody M.4.1.1. on MYBPC3 | |
|---|---|
| Epitope regions found on MYBC3 | AA (from - to) |
| -VAA- | 21-23 |
| -QRGGSDI- (SEQ ID NO: 21) / -SNK- | 43-49 / 52-54 |
| -VREVGPADQGSYAV- (SEQ ID NO: 22) | 68-81 |

Conclusion: the selected antibodies 7B5 and M-4.1.1 do recognize non-linear, conformational epitopes presented by the natively folded MYBPC3 protein.

**Example 7: Elecsys Assay**

Biomarker measurement (Exemplary method for the detection of MYBPC3)

Serum and plasma concentrations of the biomarker MYBPC3 were measured with the commercialized MYBPC3 Elecsys® reagents from Roche Diagnostics (Mannheim, Germany). The biomarker MYBPC3 was measured with prototype Elecsys® reagents from Roche Diagnostics (Mannheim, Germany).

MYBPC3-Assay using biotinylated rabbit MAB<MYBPC3>rK/M-7B5-F(ab')2-Bi and ruthenylated MAB<MYBPC3>rH-4.1.1-IgG-P8-sulfoRu. An electrochemiluminescence immunoassay (ECLIA) for the specific measurement of MYBPC3 in particular in human serum or plasma samples was developed using the Elecsys® cobas analyzer e601. The Elecsys MYBPC3 immunoassay is an electrochemiluminescence immunoassay (ECLIA) that functions via the sandwich principle. There are two antibodies included in the assay, namely a biotinylated monoclonal antibody F(ab')2-fragment MAB<MYBPC3> (MAB<MYBPC3_1-330>Bi; capture antibody, such as 7B5) and a ruthenylated monoclonal anti-MYBPC3 antibody MAB<MYBPC3> (MAB<MYBPC3_1-330>-Ru; detection antibody, such as 4.1.1), which form sandwich immunoassay complexes with MYBPC3 in the sample. The complexes are then bound to solid-phase streptavidin-coated microparticles. These are captured magnetically onto the electrode surface, leading to chemiluminescence emission upon application of voltage to the electrode, which is measured by a photomultiplier. Results are determined via an instrument-specific calibration curve determined by series of 5 calibrators with different concentrations of MYBPC3 across the measuring range. Samples are measured applying assay protocol 2 with pipetting volumes of 50 ul of the sample, 60 ul of reagent 1 (R1), 60 $\mu$l of reagent 2 (R2) and 30 $\mu$l of magnetic beads. R1 is containing MAB<MYBPC3>F(ab')2-Bi in phosphate reaction buffer and reagent 2 (R2) is containing MAB<MYBPC3>-Ru in the same reaction buffer.

**Example 8: Assay performance: Head-to-Head comparison Roche-Erenna**

To evaluate the clinical utility of our in-house developed MYBPC3 assay, we compared our assay performance to the performance of the established high-sensitivity MYBPC3 (alias MYBPC3) Assay on the Erenna platform available by Millipore Sigma. Performance of said assay was published for the clinical studies BASEL V (Kozhuharov et al. (2021)) and APACE (Kaier et al. (2022)). The BASEL V study was designed to enable the rapid triage of patients with suspected Acute Heart Failure (AHF) while the APACE study focused on providing accelerated r ule-out and r ule-in decisions in case of Myocardial Infarction (MI).

Erenna - Roche comparison on BASEL V dataset:

Data: The head-to-head comparison between the MYBPC3 Roche Assay and MYBPC3 Erenna Assay is based on the BASEL V data set. Publication Basel V study with analysis using Erenna assay: Kozhuharov et al. Eur J Heart Fail. 2021 May;23(5):716-725. doi: 10.1002/ejhf.2094. Epub 2021 Feb 5. PMID: 33421273.

**Outcome correlation:**

For in total 837 patients, we have matched Roche and Erenna MYBPC3 measurements. The correlation between the two assays is high (log-transformed, pearson correlation = 0.894). The results are shown in Figure 6.

**Outcome clinical performance:**

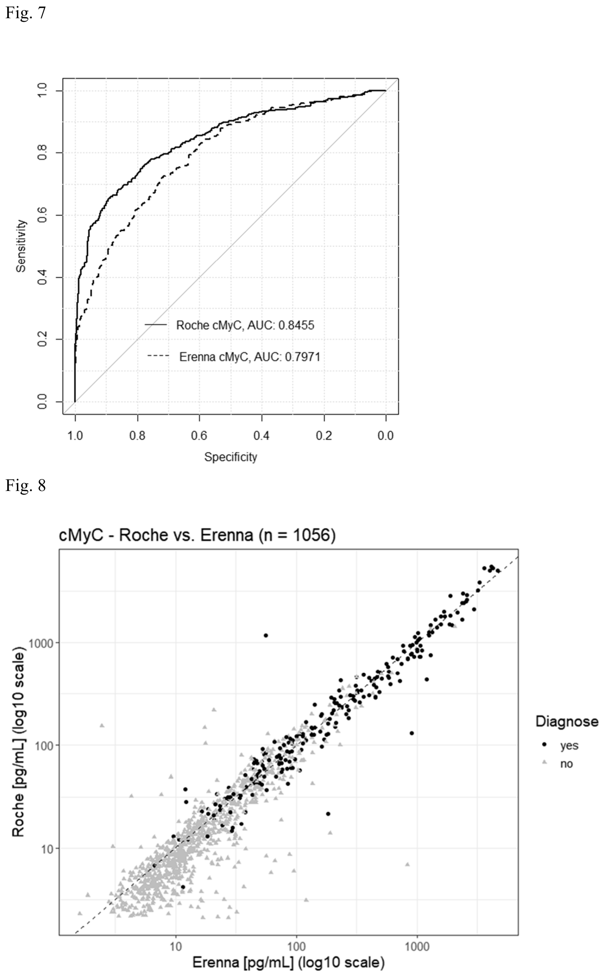

Diagnosis of heart failure: For this analysis we have 837 unique patients, 444 patients with heart failure. The AUC of the Roche MYBPC3 Assay is higher compared to the Erenna Assay (see plot). Using a one sided DeLong test to compare the AUC's shows high significance (p-value = 7.878e-09). The results are shown in Figure 7.

Erenna - Roche comparison on APACE dataset:

Data: The head-to-head comparison between the MYBPC3 Roche Assay and MYBPC3 Erenna Assay is based on the APACE data set. Publication APACE study with analysis using Erenna assay: Kaier et al., A 0/1h-algorithm using cardiac myosin-binding protein C for early diagnosis of myocardial infarction, European Heart Journal. Acute Cardiovascular Care, Volume 11, Issue 4, April 2022, Pages 325-335,

Outcome correlation: For in total 1056 patients we have matched Roche and Erenna MYBPC3 measurements. The correlation between the two assays is high (Pearson correlation = 0.9726981). The results are shown in Fig. 8.

Outcome clinical performance:

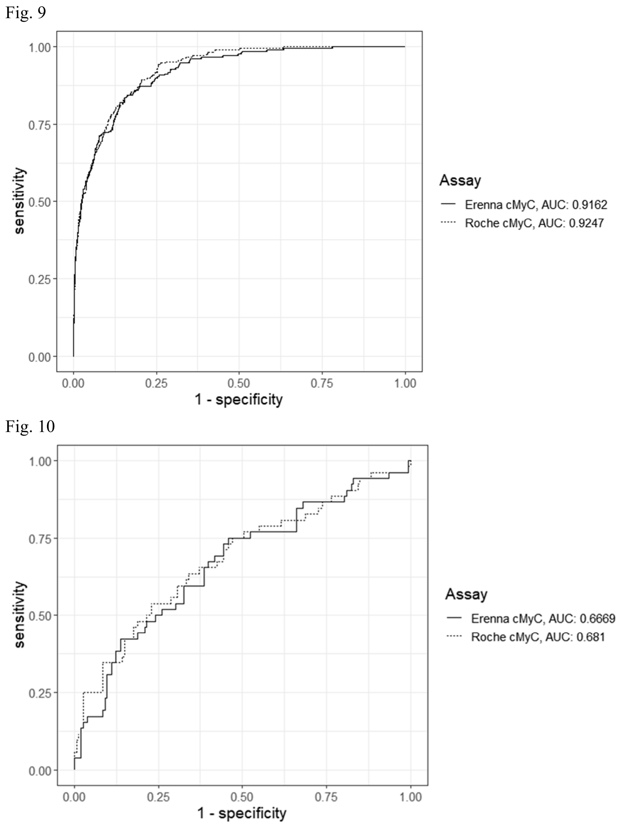

1) Diagnosis of MI: For this analysis we have 992 unique patients, 205 patients with an MI (NSTEMI and STEMI). The AUC of the Roche MYBPC3 Assay is higher compared to the Erenna Assay. Using a one sided DeLong test to compare the AUC's shows significance (p-value = 0.02269). Similar performance in the rule in (left-bottom) and slightly better performance in the rule out (right-up) zone was observed. The results are shown in Fig. 9.

2) Distinguishing between Type 1 and Type 2 MI: In total we have 52 Type 2 and 153 Type 1. The AUC is higher for the Roche Assay. The DeLong test shows no significance (p-value = 0.1387). The results are shown in Figure 10.

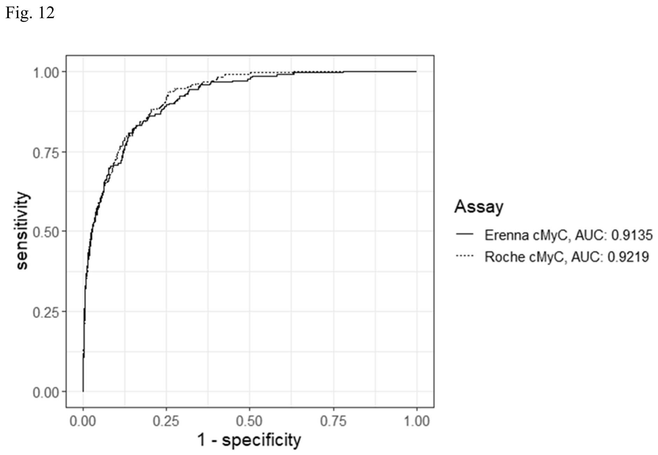

3) Risk prediction for Major Adverse Cardiovascular Events (MACE) within 30 days: This analysis is based on 986 patients with follow-up data over 30 days after presentation to the emergency department. 204 patients had an MI as index event (NSTEMI and STEMI), 3 patients hat an MI during follow-up and 2 patients died (cardiovascular and non-cardiovascular deaths have been considered). The AUC of the Roche MYBPC3 Assay is higher compared to the Erenna Assay. Using a one-sided DeLong test to compare the AUC's shows significance (p-value = 0.0232). Similar performance in the rule-in (left-bottom) and slightly better performance in the rule-out (right-up) zone was observed. The results are shown in Fig. 12.

**Claims**

1. A monoclonal antibody, or fragment thereof, which binds to MYBPC3 (Myosin binding protein C, cardiac type), comprising

    (a) a light chain variable domain comprising

        (a1) a light chain CDR1 having an amino acid sequence as shown in SEQ ID NO: 9 (RASQNIGTNMH),
        (a2) a light chain CDR2 having an amino acid sequence as shown in SEQ ID NO: 10 (FASESFS), and
        (a3) a light chain CDR3 having an amino acid sequence as shown in SEQ ID NO: 11 (QQSYNWPFT);

    and
    (b) a heavy chain variable domain comprising

        (b1) a heavy chain CDR1 having an amino acid sequence as shown in SEQ ID NO: 12 (GYGVN),
        (b2) a heavy chain CDR2 having an amino acid sequence as shown in SEQ ID NO: 13 (MIWGDGSTDYNSVYKS), and
        (b3) a heavy chain CDR3 having an amino acid sequence as shown in SEQ ID NO: 14 (RDTGGASMDY).

2. The monoclonal antibody or fragment thereof of claim 1, wherein said light chain variable domain is at least 85%, such as at least 90% or at least 95% identical to the light chain variable domain having a sequence as shown in SEQ ID NO: 15 (DILMTQSPAILSVSPGERVSFSCRASQNIGTNMHWYQQRTNGSPRLLIRFAS ESFSGIPSRFSGTGSGTDFTL RINSVESEDIADYYCQQSYNWPFTFGAGTKLE LK), and said heavy chain variable domain is at least 85%, such as at least 90% or at least 95% identical to the heavy chain variable domain having a sequence as shown in SEQ ID NO: 16 (QVQLKESGPGLVAPSQSLSITCTVSGFSLTGYGVN WVRQPPGKGLEWLGMIWGDGSTDYNSVYKSRLSISK DNSKSQVFLKMNSL QTDDTARYYCARRDTGGASMDYWGQGTSVTVSS).

3. The monoclonal antibody or fragment of claim 1 or 2, wherein the light chain variable domain comprises a sequence as shown in SEQ ID NO: 15, and the heavy chain variable domain comprises a sequence as shown in SEQ ID NO: 16.

4. The monoclonal antibody, or fragment thereof, of any one of claims 1 to 3, wherein the antibody, or fragment thereof, is labelled, and/or wherein the fragment is a Fab fragment, a Fab' fragment, a Facb fragment, a F(ab')2 fragment, a scFv fragment, or a Fv fragment, in particular wherein the fragment is a F(ab')2 fragment.

5. *In vitro* use of the monoclonal antibody, or fragment thereof, of any one of claims 1 to 4, for the detection of MYBPC3 in a sample, for example wherein the detection is the quantitative detection.

6. The *in vitro* use of claim 5, wherein the sample is a body fluid, such as a blood, serum or plasma sample, in particular wherein the sample is a human blood, serum or plasma sample.

7. A method for detecting MYBPC3 in a sample, comprising

   (a) contacting a sample comprising MYBPC3 with the monoclonal antibody, or fragment thereof, of any one of claims 1 to 4, thereby forming a complex comprising MYBPC3 and said monoclonal antibody, or fragment thereof, and
   (b) detecting the complex formed in step (a), thereby detecting MYBPC3 in said sample.

8. A complex comprising

   i) MYBPC3, and
   ii) the monoclonal antibody, or fragment thereof, of any one of claims 1 to 4.

9. *In vitro* use of the monoclonal antibody, or fragment thereof, of any one of claims 1 to 4 for assessing a cardiac condition.

10. The *in vitro* use of claim 9, wherein the cardiac condition is heart failure, or wherein the cardiac condition is myocardial injury or myocardial infarction.

11. The *in vitro* use of claim 10, wherein the cardiac condition is myocardial infarction, and wherein

   ● myocardial infarction is ruled out,
   ● it is differentiated between Type 1 and Type 2 myocardial infarction, and/or
   ● the risk for myocardial infarction and/or cardiovascular death for patients suspected of acute coronary syndrome (ACS) is predicted, for example the risk at 30 days.

Fig. 1

Fig. 2

(a)                          (b)                              (c)                    (d)

Fig. 3
A)

B)

Fig. 4

A)

B)

Fig. 5

Fig. 6

Fig. 7

Fig. 8

Fig. 9

Fig. 10

Fig. 11

>MAB 7B5 Variable heavy

QEQLEESGGGLVKPGGTLTLTCKVSGFDFS **SGYDMC** WVRQAPGKGLESIA
**CIGSSSASTWYANWVNG** RFTVSRSTSLNTVDLKMTSLTVADTATYFCAR **ESSTEYYYNL**
WGPGTLVTVSS (SEQ ID NO: 8)


>MAB 7B5 Variable light

DIVMTQTPASVEAAVGGTVTIKC **QASQSIGNALA** WYQQKPGQPPKLLIY **STSYLPS**
GVPSRFKGGGSGAEYTLTISDLECADAATYYC **QSYYVSTSSSDRSS** FGGGTEVVVK
(SEQ ID NO: 7)


>MAB 4.1.1 Variable heavy

QVQLKESGPGLVAPSQSLSITCTVSGFSLT **GYGVN** WVRQPPGKGLEWLG
**MIWGDGSTDYNSVYKS** RLSISKDNSKSQVFLKMNSLQTDDTARYYCAR **RDTGGASMDY**
WGQGTSVTVSS (SEQ ID NO: 16)

>MAB 4.1.1 Variable light

DILMTQSPAILSVSPGERVSFSC **RASQNIGTNMH** WYQQRTNGSPRLLIR **FASESFS**
GIPSRFSGTGSGTDFTLRINSVESEDIADYYC **QQSYNWPFT** FGAGTKLELK (Seq ID
NO: 15)

Fig. 12

Fig. 13

Epitope regions of antibody M.4.1.1.

MPEPGKKPVSAFSKKPRSVE**VAA**GSPAVFEAETERAGVKVRW**QRGGSDI**SA**SNK**YGLATEGT
RHTLT**VREVGPADQGSYAV**IAGSSKVKFDLKVIEAEKAEPMLAPAPAPAEATGAPGEAPAPA
AELGESAPSPKGSSSAALNGPTPGAPDDPIGLFVMRPQDGEVTVGGSITFSARVAGASLLKP
PVVKWFKGKWVDLSSKVGQHLQLHDSYDRASKVYLFELHITDAQPAFTGSYRCEVSTKDKFD
CSNFNLTVHEAMGTGDLDLLSAFRRTSLAGGGRRISDSHEDTGILDFSSLLKKRDSFRTPRD
SKLEAPAEEDVWEILRQAPPSEYERIAFQYGVTDLRGMLKRLKGMRRDEKKSTAFQKKLEPA
YQVSKGHKIRLTVELADHDAEVKWLKNGQEIQMSGSKYIFESIGAKRTLTISQCSLADDAAY
QCVVGGEKCSTELFVKEPPVLITRPLEDQLVMVGQRVEFECEVSEEGAQVKWLKDGVELTRE
ETFKYRFKKDGQRHHLIINEAMLEDAGHYALCTSGGQALAELIVQEKKLEVYQSIADLMVGA
KDQAVFKCEVSDENVRGVWLKNGKELVPDSRIKVSHIGRVHKLTIDDVTPADEADYSFVPEG
FACNLSAKLHFMEVKIDFVPRQEPPKIHLDCPGRIPDTIVVVAGNKLRLDVPISGDPAPTVI
WQKAITQGNKAPARPAPDAPEDTGDSDEWVFDKKLLCETEGRVRVETTKDRSIFTVEGAEKE
DEGVYTVTVKNPVGEDQVNLTVKVIDVPDAPAAPKISNVGEDSCTVQWEPPAYDGGQPILGY
ILERKKKKSYRWMRLNFDLIQELSHEARRMIEGVVYEMRVYAVNAIGMSRPSPASQPFMPIG
PPSEPTHLAVEDVSDTTVSLKWRPPERVGAGGLDGYSVEYCPEGCSEWVAALQGLTEHTSIL
VKDLPTGARLLFRVRAHNMAGPGAPVTTTEPVTVQEILQRPRLQLPRHLRQTIQKKVGEPVN
LLIPFQGKPRPQVTWTKEGQPLAGEEVSIRNSPTDTILFIRAARRVHSGTYQVTVRIENMED
KATLVLQVVDKPSPPQDLRVTDAWGLNVALEWKPPQDVGNTELWGYTVQKADKKTMEWFTVL
EHYRRTHCVVPELIIGNGYYFRVFSQNMVGFSDRAATTKEPVFIPRPGITYEPPNYKALDFS
EAPSFTQPLVNRSVIAGYTAMLCCAVRGSPKPKISWFKNGLDLGEDARFRMFSKQGVLTLEI
RKPCPFDGGIYVCRATNLQGEARCECRLEVRVPQ **(SEQ ID NO: 23)**

Fig 14

Epitope regions of antibody 7B5

MPEPGKKPVSAFSKKPRSVEVAAGSPAVFEAE**TERAGVKV**RWQRGGSDISASNK**YGLATEGTRHTL**TV
REVGPADQGSYAVIAGSSKVKFDLKVIEAEKAEPMLAPAPAPAEATGAPGEAPAPAAELGESAPSPKG
SSSAALNGPTPGAPDDPIGLFVMRPQDGEVTVGGSITFSARVAGASLLKPPVVKWFKGKWVDLSSKVG
QHLQLHDSYDRASKVYLFELHITDAQPAFTGSYRCEVSTKDKFDCSNFNLTVHEAMGTGDLDLLSAFR
RTSLAGGGRRISDSHEDTGILDFSSLLKKRDSFRTPRDSKLEAPAEEDVWEILRQAPPSEYERIAFQY
GVTDLRGMLKRLKGMRRDEKKSTAFQKKLEPAYQVSKGHKIRLTVELADHDAEVKWLKNGQEIQMSGS
KYIFESIGAKRTLTISQCSLADDAAYQCVVGGEKCSTELFVKEPPVLITRPLEDQLVMVGQRVEFECE
VSEEGAQVKWLKDGVELTREETFKYRFKKDGQRHHLIINEAMLEDAGHYALCTSGGQALAELIVQEKK
LEVYQSIADLMVGAKDQAVFKCEVSDENVRGVWLKNGKELVPDSRIKVSHIGRVHKLTIDDVTPADEA
DYSFVPEGFACNLSAKLHFMEVKIDFVPRQEPPKIHLDCPGRIPDTIVVVAGNKLRLDVPISGDPAPT
VIWQKAITQGNKAPARPAPDAPEDTGDSDEWVFDKKLLCETEGRVRVETTKDRSIFTVEGAEKEDEGV
YTVTVKNPVGEDQVNLTVKVIDVPDAPAAPKISNVGEDSCTVQWEPPAYDGGQPILGYILERKKKKSY
RWMRLNFDLIQELSHEARRMIEGVVYEMRVYAVNAIGMSRPSPASQPFMPIGPPSEPTHLAVEDVSDT
TVSLKWRPPERVGAGGLDGYSVEYCPEGCSEWVAALQGLTEHTSILVKDLPTGARLLFRVRAHNMAGP
GAPVTTTEPVTVQEILQRPRLQLPRHLRQTIQKKVGEPVNLLIPFQGKPRPQVTWTKEGQPLAGEEVS
IRNSPTDTILFIRAARRVHSGTYQVTVRIENMEDKATLVLQVVDKPSPPQDLRVTDAWGLNVALEWKP
PQDVGNTELWGYTVQKADKKTMEWFTVLEHYRRTHCVVPELIIGNGYYFRVFSQNMVGFSDRAATTKE
PVFIPRPGITYEPPNYKALDFSEAPSFTQPLVNRSVIAGYTAMLCCAVRGSPKPKISWFKNGLDLGED
ARFRMFSKQGVLTLEIRKPCPFDGGIYVCRATNLQGEARCECRLEVRVPQ **(SEQ ID NO: 23)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015110114 A
- WO 2015110115 A
- WO 2012107419 A
- US 4016043 A
- US 4424279 A
- US 4018653 A
- WO 215110115 A

### Non-patent literature cited in the description

- **THYGESEN K et al.** *Circulation*, 2018, vol. 138, e618-e651
- **NESTELBERGER et al.** *JAMA Cardiol.*, 2021
- **KOZHUHAROV et al.** *Eur J Heart Fail.*, May 2021, vol. 23 (5), 716-725
- **KAIER et al.** *European Heart Journal. Acute Cardiovascular Care*, April 2022, vol. 11 (4), 325-335
- **MARJOT et al.** *C. Transl Res*, 2016, vol. 170, 17-25. e5
- **SMITH** ; **WATERMAN**. *Add. APL. Math.*, 1981, vol. 2, 482
- **NEEDLEMAN** ; **WUNSCH**. *J. Mol. Biol.*, 1970, vol. 48, 443
- **PEARSON** ; **LIPMAN**. *Proc. Natl. Acad. Sci. (USA)*, 1988, vol. 85, 2444
- **NEEDLEMAN**. *J. Mol. Biol.*, 1970, vol. 48, 444-453
- **RICE, P** ; **LONGDEN, I** ; **BLEASBY, A**. EMBOSS: The European Molecular Biology Open Software Suite. *Trends in Genetics*, 2000, vol. 16 (6), 276-277
- Pierce Catalog and Handbook. Pierce Chemical Co., 1994, 111
- **KUBY, J.** Immunology. W.H. Freeman & Co, 1997
- Sequences of Proteins of Immunological Interest. **KABAT et al.** US Dept. of Health and Human Services, PHS, NIH, NIH. 1991
- **LEFRANC, M.P. et al.** *Dev Comp Immunol.*, 2003, vol. 27 (1), 55-77
- **RICHTER, M.M.** *Chem. Rev.*, 2004, vol. 104, 3003-3036
- **THYGESEN et al.** *Eur J Heart*, 2019, vol. 40 (3), 237-269
- **KAIER et al.** A 0/1h-algorithm using cardiac myosin-binding protein C for early diagnosis of myocardial infarction. *European Heart Journal. Acute Cardiovascular Care*, April 2022, vol. 11 (4), 325-335